(19) Europäisches Patentamt
European Patent Office
Office européen des brevets

(11) Publication number: **0 008 154**
**A1**

# (12) EUROPEAN PATENT APPLICATION

(21) Application number: 79301036.4

(22) Date of filing: 01.06.79

(51) Int. Cl.³: **C 07 C 143/675**, C 07 C 143/80, A 61 K 31/18, A 61 K 31/185 // C07C79/46, C07C143/55, C07C143/70

(30) Priority: 11.07.78 US 923742
11.07.78 US 923743
11.07.78 US 923744
11.07.78 US 923745
11.07.78 US 923746

(43) Date of publication of application: 20.02.80 Bulletin 80/4

(84) Designated Contracting States: AT BE CH DE FR GB IT LU NL SE

(71) Applicant: **AMERICAN CYANAMID COMPANY, Berdan Avenue, Wayne New Jersey 06904 (US)**

(72) Inventor: **Siuta, Gerald Joseph, 14, Georgetown Oval, New City New York, 10956 (US)**
Inventor: **Poletto, John Frank, 35 Nanuet Avenue, Nanuet New York (US)**
Inventor: **Conrow, Ransom Brown, 50 Lt. Cox Drive, Pearl River New York (US)**
Inventor: **Bernstein, Seymour, 26 Scott Drive, New City New York (US)**

(74) Representative: **Allam, Peter Clerk et al, TREGEAR, THIEMANN & BLEACH Enterprise House Isambard Brunel Road, Portsmouth, Hants PO1 2AN (GB)**

## (54) Ureylene phenylene anionic naphthalene-sulfonic acids, their use as complement system inhibitors in a body fluid and process for their preparation.

(57) The present invention provides novel ureylene phenylene anionic naphthalenesulfonic acids, which have complement-inhibiting properties and which may be represented by the general formula:

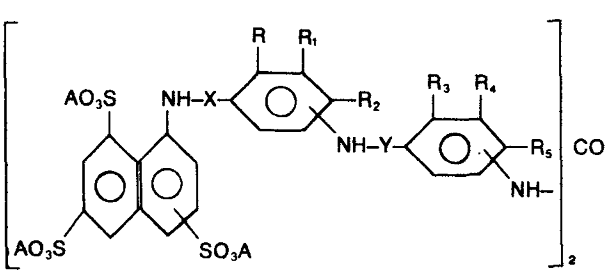

wherein X and Y are each selected from the group consisting of –CO– and –$SO_2$–; R and $R_3$ are each selected from the group consisting of hydrogen, methyl and –$SO_3$A, wherein A is a pharmaceutically acceptable salt cation; $R_1$ and $R_4$ are each selected from the group consisting of hydrogen and –COOB, wherein B is selected from the group consisting of hydrogen and a pharmaceutically acceptable salt cation; $R_2$ and $R_5$ are each selected from the group consisting of hydrogen and methyl; with the proviso that R, $R_1$, $R_2$, $R_3$, $R_4$ and $R_5$ may not all be hydrogen; with the second proviso that when X and Y are both –CO– and $R_2$ is methyl, then R, $R_1$, $R_3$, $R_4$ and $R_5$ may not all be hydrogen; and with the third proviso that no phenyl moiety can contain both –$SO_3$A and –COOB.

The corresponding amine compounds of the formula below are novel intermediates for the novelureylene compounds above:

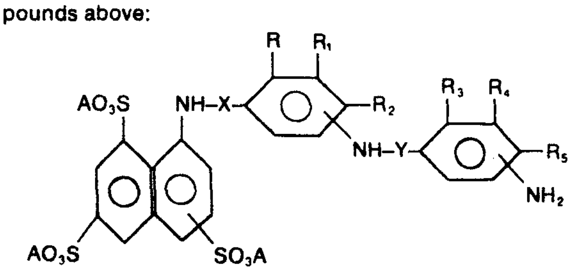

EP 0 008 154 A1

0008154

26,913

TITLE MODIFIED
see front page

TITLE

UREYLENE PHENYLENE ANIONIC NAPHTHALENESULFONIC ACIDS

The term "complement" refers to a complex group of proteins in body fluids that, working together with antibodies or other factors, play an important role as mediators of immune, allergic, immunochemical and/or immunopathological reactions. The reactions in which complement participates take place in blood serum or in other body fluids, and hence are considered to be humoral reactions.

With regard to human blood, there are at present more than 11 proteins in the complement system. These complement proteins are designated by the letter C and by number: C1, C2, C3 and so on up to C9. The complement protein C1 is actually an assembly of subunits designated C1q, C1r and C1s. The numbers assigned to the complement proteins reflect the sequence in which they become active, with the exception of complement protein C4, which reacts after C1 and before C2. The numerical assignments for the proteins in the complement system were made before the reaction sequence was fully understood. A more detailed discussion of the complement system and its role in the body processes can be found in, for example, Bull. World Health Org., 39, 935-938 (1968); Ann. Rev. Medicine, 19, 1-24 (1968); The John Hopkins Med. J., 128, 57-74 (1971); Harvey Lectures, 66, 75-104 (1972); The New England Journal of Medicine, 287, 452-454; 489-495; 545-549; 592-596; 642-646 (1972); Scientific American 229, (No. 5), 54-66 (1973); Federation Proceedings, 32, 134-137 (1973); Medical World News, October 11, 1974, pp. 53-58; 64-66; J. Allergy Clin. Immunol., 53, 298-302 (1974); Cold Spring Harbor

Conf. Cell Prolifieration 2/Proteases Biol. Control/229-241 (1975); Annals of Internal Medicine, 84, 580-593 (1976); "Complement: Mechanisms and Functions", Prentice-Hall, Englewood Cliffs, N. J. (1976).

The complement system can be considered to consist of three sub-systems: (1) a recognition unit (C1q) which enables it to combine with antibody molecules that have detected a foreign invader; (2) an activation unit (C14, C1s, C2, C4, C3) which prepared a site on the neighboring membrane; and (3) an attack unit (C5, C6, C7, C8 and C9) which creates a "hole" in the membrane. The membrane attack unit in non--specific; it destroys invaders only because it is generated in their neighborhood. In order to minimize damage to the host's own cells, its activity must be limited in time. This limitation is accomplished partly by the spontaneous decay of activated complement and partly by interference by inhibitors and destructive enzymes. The control of complement, however, is not perfect, and there are times when damage is done to the host's cells. Immunity is, therefore, a double-edged sword.

Activation of the complement system also accelerates blood clotting. This action comes about by way of the complement-mediated release of a clotting factor from platelets. The biologically active complement fragments and complexes can become involved in reactions that damage the host's cells, and these pathogenic reactions can result in the development of immune-complex diseases. For example, in some forms of nephritis, complement damages the basal membrane of the kidney, resulting in the escape of protein from the blood into the urine. The disease disseminated lupus erythematosus belongs in this category; its symptoms include nephritis, visceral lesions and skin eruptions. The treatment of diphtheria or tetanus with the injection of large amounts of antitoxin sometimes results in serum sickness, an immune-complex disease. Rheumatoid arthritis also involves immune complexes. Like disseminated lupus erythematosus, it is an autoimmune disease in which the disease symptoms are caused by pathological effects of the immune system in the host's tissues. In summary,

the complement system has been shown to be involved with inflammation, coagulation, fibrinolysis, antibody-antigen reactions and other metabolic processes.

In the presence of antibody-antigen complexes the complement proteins are involved in a series of reactions which may lead to irreversible membrane damage if they occur in the vicinity of biological membranes. Thus, while complement constitutes a part of the body's defense mechanism against infection it also results in inflammation and tissue damage in the immunopathological process. The nature of certain of the complement proteins, suggestions regarding the mode of complement binding to biological membranes and the manner in which complement effects membrane damage are discussed in Annual Review in Biochemistry, 38, 389 (1969).

A variety of substances have been disclosed as inhibiting the complement system, i.e., as complement inhibitors. For example, the compounds 3,3'-ureylenebis-[6-(2--amino-8-hydroxy-6-sulfo-1-naphthylzao)]benzenesulfonic acid, tetrasodium salt (chlorazol fast pink), heparin and a sulphated dextran have been reported to have an anticomplementary effect, British Journal of Experimental Pathology, 33, 327-339 (1952). German Patent No. 2,254,893 or South African Patent No. 727,923 discloses certain 1-(diphenylmethyl)-4-(3-phenylallyl)piperazines useful as complement inhibitors. Other chemical compounds having complement inhibiting activity are disclosed in, for example, Journal of Medicinal Chemistry, 12, 415-419; 902-905; 1049-1052; 1053-1056 (1969); Canadian Journal of Biochemistry, 47, 547-552 (1969); The Journal of Immunology, 93, 629-640 (1964); The Journal of Immunology, 104, 279-288 (1970); The Journal of Immunology, 106, 241-245 (1971); and The Journal of Immunology, 111, 1061-1066 (1973). Biochim. Biophys. Acta, 317, 539-548 (1973); Life Sciences, 13, 351-362 (1973); Journal of Immunology, 113, 584 (1974); Immunology, 26, 819-829 (1974); Journal of Medicinal Chemistry, 17, 1160-1167 (1974); Biochim. Biophys. Res. Comm., 67, 225-263 (1975); Ann. N. Y. Acad. Sci., 256, 441-450 (1975); Journal

of Medicinal Chemistry, 19, 634-639, 1079 (1976); Journal of Immunology, 118, 466 (1977); Arch. Int. Pharmacodyn., 226, 281-285 (1977); Biochem. Pharmacol. 26, 325-329 (1977); Journal Pharm. Sci., 66, 1367-1377 (1977); Chem. Pharm. Bull., 25, 1202-1208 (1977); Biochim. Biophys. Acta, 484, 417-422 (1977) and Journal Clin. Microbiology, 5, 278-284 (1977).

It has been reported that the known complement inhibitors epsilon-aminocaproic acid and tranexamic acid have been used with success in the treatment of hereditary angioneurotic edema, a disease state resulting from an inherited deficiency or lack of function of the serum inhibitor of the activated first component of complement (C1 inhibitor), The New England Journal of Medicine, 286, 808-812 (1972), 287, 452-454 (1972); Ann. Intern. Med., 84, 580-593 (1976); J. Allergy and Clin. Immunology, 60, 38-40 (1977).

It has also been reported that the drug pentosan-polysulfoester has an anticomplementary activity on human serum, both *in vitro* and *in vivo*, as judged by the reduction in total hemolytic complement activity; Pathologie Biologie, 25, 33-36, 25 (2), 105-108, 25 (3), 179-184 (1977).

It is known that the compound Suramin is moderately active as a complement inhibitor, and possess the structure:

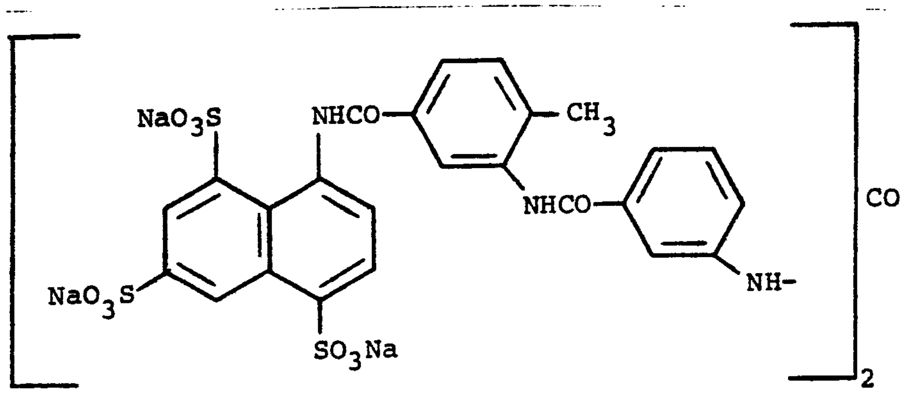

It now has been discovered that certain modifications of this structure provide compounds with enhanced inhibitory activity. This invention is based on such modifications.

The following publications, pertaining to the chemistry of Suramin, are related to the preparation of the novel compounds of this invention:

Bayer & Co., D.R.P. 278,122, June 22, 1913 [C.A. 9, 1096(1915)]

Bayer & Co., D.R.P. 288,272, Jan. 23, 1914 [C.A. 10, 2279(1916)]

Bayer & Co., D.R.P. 288,273, Feb. 21, 1914 [C.A. 10, 2279(1916)]

Frdl. 12, 185-186, 191-195 (1914-1916)

Danish Patent 20,743 (1915)

Austrian Patent 72,298 (1916)

Austrian Patent 72,303 (1916)

U. S. Patent 1,218,654 (1917)

U. S. Patent 1,218,655 (1917)

Austrian Patent 73,381 (1917)

U. S. Patent 1,308,071 (1919)

E. Fourneau, J. Tréfouel, Mme. J. Tréfouel and J. Vallee, Acad. Sci. Comp. Rend., 178, 675-676 (1924)

E. Fourneau, F. Tréfouel and J. Vallee, Ann. de L'Institut Pasteur, 38 (2), 81-114 (1924)

B. Heymann, Zeitschrift Ang. Chem., 37, 585-589 (1924)

British Patent 224,849 (1925)

U. S. Patent 1,606,624 (1926)

J. E. R. McDonagh, Brit. Med. J., 693-696 (1926) [Chem. Zentralblatt, 1769-1770 (1926 II)]

W. Roehl, Arch. Schiff. Trop. Hyg., 30 (1), 103-111 (1926)

Poulenc Fréres, D.R.P. 427,857, April 20, 1926 [Frdl. 15, 1434-1436(1928)]

I. E. Balaban and H. King, J. Chem. Soc., 3068-3097 (1927)

H. Bauer and J. Becker, Arb. Staatsinst. Exptl. Therap., 16 pp. (1928)

U. S. Patent 1,968,820 (1934)

O. Yu. Magidson, O. S. Madaeva and M. V. Rubtzov, Khim. Farm. Prom., 2, 89-94 (1935) [C.A., 30, 4492 (1936)]

U. S. 2,126,180 (1938)

P. Pratsi and L. Raffa, Farmaco Sci e Tec (Pavia), 1, 21-34 (1946)

A. Spinks, Biochem. J., 42, 109-116 (1948)

E. D. Wills and A. Wormall, Biochem. J., 47, 158-170 (1950)

German Patent 890,952 (1953) [C. A. 52, 14693 (1958)]

A. Adams, J. N. Ashley and H. Bader, J. Chem. Soc., 3739-3744 (1956) [C. A. 51, 4375i]

Publications related to the biological use of Suramin compounds for the purpose of inhibiting the complement system, including humans, as determined by the in vivo and in vitro testing of the blood serum of warm-blooded animals are:

B. Stuber and K. Lang, Arch. Exptl. Path. Pharmacol., 154, 41-49 (1930) [C. A. 25, 3067(1931)]

F. Klopstock, Zeitschrift für Immunitatsforschung und experimentalle Therapie, 75, 348-354 (1932)

H. J. Schmid, Schweiz. Med. Woch., 96, 1267-1269 (1966)

K. Lauenstein, Bayer-Symposium I, 25-30 (1969)

J. S. C. Fong and R. A. Good, Clin. Exp. Immunol., 10, 127-138 (1972)

V. Eisen and C. Loveday, Br. J. Pharmac., 49, 678-687 (1973)

D. Brackertz and F. Kueppers, Allergol. Et. Immunopath., 11, 163-168 (1974)

E. Raepple, H-U. Hill and M. Loos, Immunochemistry, 13 (3), 251-255 (1976)

This invention is concerned with ureylenebis[substituted-phenylenecarbonyl(and sulfonyl) imino-substituted--phenylenecarbonyl(and sulfonyl) imino-naphthalenetrisulfonic acids] and all pharmaceutically acceptable salts thereof, having complement inhibiting activity, which are new compounds of the general formula:

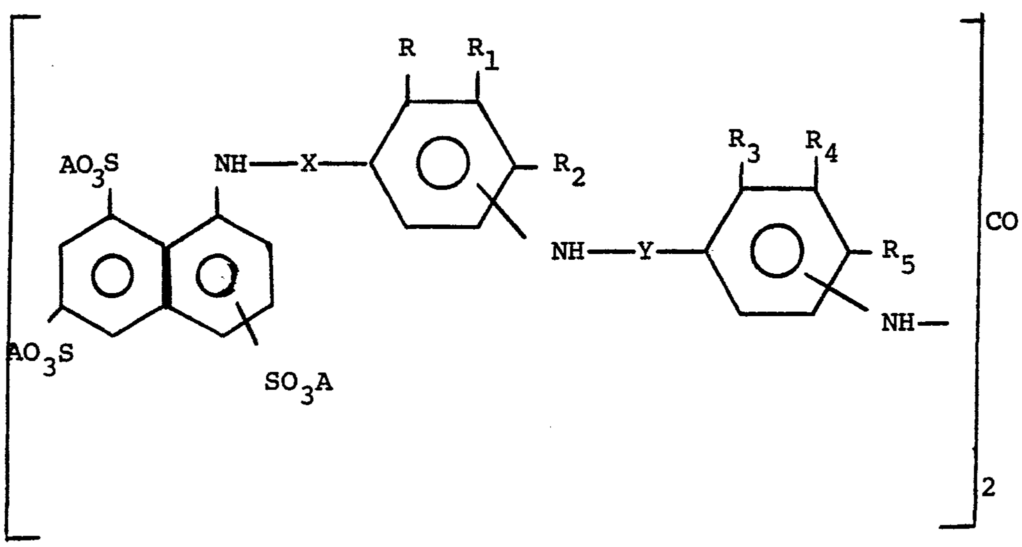

wherein X and Y are each selected from the group consisting of -CO- and $-SO_2-$; R and $R_3$ are each selected from the group consisting of hydrogen, methyl and $-SO_3A$, wherein A is a pharmaceutically acceptable salt cation; $R_1$ and $R_4$ are each selected from the group consisting of hydrogen and -COOB, wherein B is selected from the group consisting of hydrogen and a pharmaceutically acceptable salt cation; $R_2$ and $R_5$ are each selected from the group consisting of hydrogen and methyl; with the proviso that R, $R_1$, $R_2$, $R_3$, $R_4$ and $R_5$ may not all be hydrogen; with the second proviso that when X and Y are both -CO- and $R_2$ is methyl, then R, $R_1$, $R_3$, $R_4$ and $R_5$ may not all be hydrogen; and with the third proviso that no phenyl moiety can contain both $-SO_3A$ and -COOB.

A preferred embodiment of this invention consists of those compounds wherein either $R_1$ or $R_4$, or both, are -COOB; and R and $R_3$ are hydrogen.

Another preferred embodiment of this invention consists of those compounds wherein either R or $R_3$, or both, are $-SO_3A$; and $R_1$ and $R_4$ are hydrogen.

This invention is also concerned with compounds of the formula:

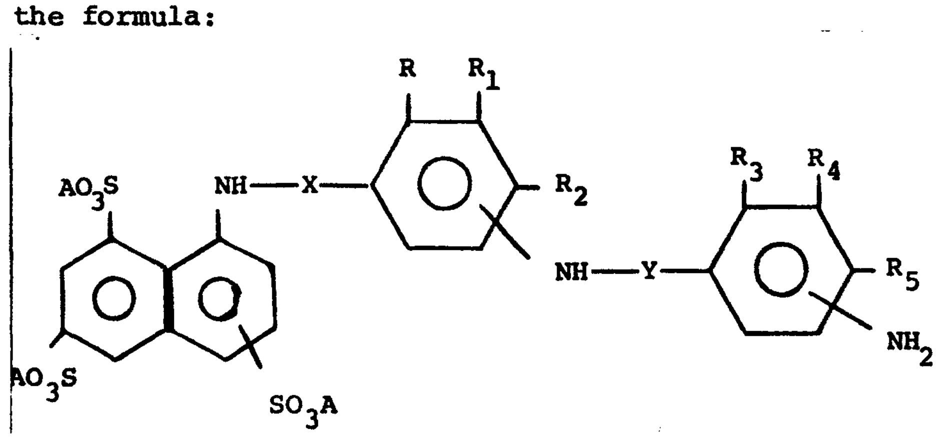

wherein X and Y are each selected from the group consisting of -CO- and $-SO_2-$; R and $R_3$ are each selected from the group consisting of hydrogen, methyl and $-SO_3A$,, wherein A is a pharmaceutically acceptable salt cation; $R_1$ and $R_4$ are each selected from the group consisting of hydrogen and -COOB, wherein B is selected from the group consisting of hydrogen

and a pharmaceutically acceptable salt cation; $R_2$ and $R_5$ are each selected from the group consisting of hydrogen and methyl; with the proviso that R, $R_1$, $R_2$, $R_3$, $R_4$ and $R_5$ may not all be hydrogen; with the second proviso that when X and Y are both -CO- and $R_2$ is methyl, then R, $R_1$, $R_3$, $R_4$ and $R_5$ may not all be hydrogen; and with the third proviso that no phenyl moiety can contain both $-SO_3A$ and -COOB; said compounds being useful as intermediates for the preparation of the complement inhibiting compounds described above. Some of the intermediate compounds also possess complement inhibiting activity.

The novel intermediate amine compounds of the invention are prepared by reacting the appropriate 8-amino--1,3,5 (and 1,3,6)-naphthalenetrisulfonic acid trialkali metal salt with a nitrobenzoyl chloride such as m-nitrobenzoyl chloride, 3-nitro-p-toluoyl chloride and 3-carbomethoxy-5--nitrobenzoyl chloride, or with a nitrosulfobenzoic acid anhydride such as 4-nitro-2-sulfobenzoic acid anhydride, or with a nitrobenzenesulfonyl chloride such as 2-methyl-5-nitrobenzenesulfonyl chloride, 4-methyl-3-nitrobenzenesulfonyl chloride, 5-nitro-2,4-xylenesulfonyl chloride and m-nitrobenzenesulfonyl chloride, for 1.5-36 hours in an aqueous solution made alkaline with alkali metal carbonate or alkali metal acetate trihydrate. After neutralization, the solution is diluted with absolute ethanol to provide the corresponding nitro-substituted--phenylenesulfonylimino-1,3,5 (and 1,3,6) naphthalenetrisulfonic acid, trialkali metal salt or the corresponding nitro-substituted phenylenecarbonylimino-1,3,5 (and 1,3,6) naphthalenetrisulfonic acid, trialkali metal salt.

Hydrogenation of the preceding nitro trialkali metal salts using 10% palladium-carbon catalyst, filtration, concentration and treatment with absolute ethanol provides the corresponding amino-substituted-phenylenesulfonylimino and amino-substituted-phenylene carbonylimino naphthalenetrisulfonic acid, trialkali metal salt compounds.

The amino compounds above, dissolved in aqueous media and made alkaline with either alkali metal hydroxide or anhydrous alkali metal carbonate are reacted once more with

the desired nitrobenzenesulfonyl chloride, nitrosulfobenzoic acid anhydride or nitrobenzoyl chloride listed above for 1.5-36 hours. After neutralization, the solution is diluted with absolute ethanol to provide the corresponding nitro--substituted-phenylenecarbonyl (and sulfonyl) imino-substituted--phenylenecarbonyl (and sulfonyl) imino-naphthalenetrisulfonic acid, trialkli metal salt.

The novel intermediate amine compounds of the invention are then obtained by hydrogenation of the above nitro compounds using 10% palladium-carbon catalyst in water as previously described, filtration and evaporation of the filtrate produces a residue which is dissolved in water and precipitated with absolute ethanol to provide the desired product.

The novel ureylene compounds of the invention, which are active complement inhibitors, are then provided by treatment of the above intermediate amine compounds with phosgene in aqueous media made alkaline with alkali metal carbonate or pyridine, neutralization and precipitation from aqueous solution with alcohol.

This invention is concerned with a method of inhibiting the complement system in a body fluid, such as blood serum, which comprises subjecting body fluid complement to the action of an effective complement inhibiting amount of a compound encompassed within the formulae hereinabove. The method of use aspect of this invention is also concerned with a method of inhibiting the complement system in a warm-blooded animal which comprises administering to said animal an effective complement inhibiting amount of a compound encompassed within the formulae hereinabove. Body fluid can include blood, plasma, serum, synovial fluid, cerebrospinal fluid, or pathological accumulations of fluid such as pleural effusion, etc.

Compounds of the present invention find utility as complement inhibitors in body fluids and as such may be used to ameliorate or prevent those pathological reactions required in the function of complement and in the therapeutic treatment of warm-blooded animals having immunologic diseases such as rheumatoid arthritis, systemic lupus erythematosus, certain kinds of glomerulonephritis, certain kinds of auto-allergic hemolytic anemia, certain kinds of platelet disorders and certain kinds of vasulitis. The compounds herein may also be used in the therapeutic treatment of warm-blooded animals having non-immunologic diseases such as paroxysmal nocturnal hemoglobinuria, hereditary antioneurotic edema (treated with Suramin, etc.) and inflammatory states induced by the action of bacterial or lysosomal enzymes on the appropriate complement components as for example, inflammation following coronary occulusion. They may also be useful in the treatment of transplant rejection and as blood culture or transport mediums.

The compounds of the present invention may be administered internally, e.g., orally or parenterally, e.g., intra--articularly, to a warm-blooded animal to inhibit complement in the body fluid of the animal, such inhibition being useful in the amelioration or prevention of those reactions dependent upon the function of complement, such as inflammatory process and cell membrane damage induced by antigen-antibody complexes. A range of doses may be employed depending on the mode of administration, the condition being treated and the particular compound being used. For example, for intravenous or subcutaneous use from about 5 to about 50 mg/kg/day, or every six hours for more rapidly excreted salts, may be used. For intra-articular use for large joints such as the knee, from about 2 to about 20 mg/joint per week may be used, with proportionally smaller doses for smaller joints. The dosage range is to be adjusted to provide optimum therapeutic response in the warm-blooded animal being treated. In general, the amount of compound administered can vary over a wide range to provide from about 5 mg/kg to about 100 mg/kg of body weight of animal per day. The usual daily dosage for a 70 kg subject may vary from about 350 mg to about 3.5 g. Unit doses of the acid or salt can contain from about 0.5 mg to about 500 mg.

While in general the sodium salts of the acids of

the invention are suitable for parenteral use, other salts may also be prepared, such as those of primary amines. e.g. ethylamine; secondary amines, e.g., diethylamine or diethanol amine; tertiary amines, e.g., pyridine or triethylamine or 2-dimethylaminomethyl-dibenzofuran; aliphatic diamines, e.g., decamethylenediamine; and aromatic diamines, can be prepared. Some of these are soluble in water, others are soluble in saline solution, and still others are insoluble and can be used for purposes of preparing suspensions for injection. Furthermore, as well as the sodium salt, those of the alkali metals, such as potassium and lithium; of ammonia; and of the alkaline earth metals, such as calcium or magnesium, may be employed. It will be apparent, therefore, that these salts embrace, in general, derivatives of salt-forming cations.

The compounds of the present invention may also be administered topically in the form of ointments, creams, lotions and the like, suitable for the treatment of complement dependent dermatological disorders.

Moreover, the compounds of the present invention may be administered in the form of dental pastes, ointments, buccal tablets and other compositions suitable for application periodrntally for the treatment of periodontitis and related diseases of the oral cavity.

In therapeutic use, the compounds of this invention may be administered in the form of conventional pharmaceutical compositions. Such compositions may be formulated so as to be suitable for oral or parenteral administration. The active ingredient may be combined in admixture with a pharmaceutically acceptable carrier, which carrier may take a wide variety of forms depending on the form of preparation desired for administration, i.e., oral or parenteral. The compounds can be used in compositions such as tablets. Here, the principal active ingredient is mixed with conventional tabletting ingredients such as corn starch, lactose, sucrose, sorbitol, talc, stearic acid, magnesium stearate, dicalcium phosphate, gums, or similar materials as non-toxic pharmaceutically acceptable diluents or carriers. The tablets or pills of the

novel compositions can be laminated or otherwise compounded to provide a dosage form affording the advantage of prolonged or delayed action or predetermined successive action of the enclosed medication. For example, the tablet or pill can comprise an inner dosage and an outer dosage component, the latter being in the form of an evenlope over the former. The two components can be separated by an enteric layer which serves to resist disintegration in the stomach and permits the inner component to pass intact into the duodenum or to be delayed in release. A variety of materials can be used for such enteric layers or coatings, such materials including a number of polymeric acids or mixtures of polymeric acids with such materials as shellac, shellac and cetyl alcohol, cellulose acetate and the like. A particularly advantageous enteric coating comprises a styrene maleic acid copolymer together with known materials contributing to the enteric properties of the coating. The tablet or pill may be colored through the use of an appropriate non-toxic dye, so as to provide a pleasing appearance.

The liquid forms in which the novel compositions of the present invention may be incorporated for administration include suitable flavored emulsions with edible oils. and the like, as well as elixirs and similar pharmaceutical vehicles. Sterile suspensions or solutions can be prepared for parenteral use. Isotonic preparations containing suitable preservatives are also desirable for injection use.

The term dosage form, as described herein, refers to physically discrete units suitable as unitary dosage for warm-blooded animal subjects. each unit containing a predetermined quantity of active component calculated to produce the desired therapeutic effect in association with the required pharmaceutical diluent, carrier or vehicle. The specification for the novel dosage forms of this invention are indicated by characteristics of the active component and the particular therapeutic effect to be achieved or the limitations inherent in the art of compounding such an active component for therapeutic use in warm-blooded animals as diclosed in this specification. Examples of suitable oral dosage forms in accord with this invention are tablets, capsules, pills, powder packets, granules, wafers. cachets. teaspoonfuls. dropperfuls, ampules. vials, segregated multiples of any of the foregoing and other forms as herein described.

The complement inhibiting activity of the compounds of this invention has been demonstrated by one or more of the following identified tests: (i) Test Code 026 (Cl inhibitor) - This test measures the ability of activated human Cl to destroy fluid phase human C2 in the presence of C4 and appropriate dilutions of the test compound. An active inhibitor protects C2 from Cl and C4; (ii) Test Code 035 (C3-C9 inhibitor) - This test determines the ability of the late components of human complement (C3-C9) to lyse EAC 142 in the presence of appropriate dilutions of the test compound. An active inhibitor protects EAC 142 from lysis by human C3-C9; (iii) Test Code 036 (C-Shunt inhibitor) - In this test human erythrocytes rendered fragile are lysed in autologous serum via the shunt pathway activated by cobra venom factor in the presence of appropriate dilutions of the test compound. Inhibition of the shunt pathway results in failure of lysis; (iv) Forssman Vasculitis Test - Here, the well known complement dependent lesion, Forssman vasculitis, is produced in guinea pigs by intradermal injection of rabbit anti-Forssman antiserum. The lesion is measured in terms of diameter, edema and hemorrhage and the extent to which a combined index of these is inhibited by prior intraperitoneal injection of the test compound at 200 mg/kg is then reported, unless otherwise stated; (v) Forssman Shock Test - Lethal shock is produced in guinea pigs by an i.v. injection of anti-Forssman antiserum and the harmonic mean death time of treated guinea pigs is compared with that of simultaneous controls; (vi) Complement Level Reduction Test - In this test, the above dosed guinea pigs, or others, are bled for serum and the complement level is determined in undiluted serum by the capillary tube method of U.S. Patent No. 3.876,376 and compared to undosed control guinea pigs: and (vii) Cap 50 Test - Here, appropriate

amounts of the test compound are added to a pool of guinea pig serum in vitro, after which the undiluted serum capillary tube assay referred to about is run. The concentration of compound inhibiting 50% is reported.

With reference to Table I, guinea pigs weighing about 300 g were dosed intravenously (i.v.) or intraperitoneally (i.p.) with 200 mg/kg of the test compound dissolved in saline and adjusted to pH 7-8. One hour after dosing the guinea pigs were decapitated, blood was collected and the serum separated. The serum was tested for whole complement using the capillary tube assay. Percent inhibition was calculated by comparison with simultaneous controls. The results appeared in Table I together with results of tests code 026, 035, 036, Cap 50, % inhibition and Forssman shock. Table I shows that the compounds of the invention possess highly significant in vitro and in vivo complement inhibiting activity in warm-blooded animals and are more active than the reference compound Suramin. Results obtained are listed in Table I.

Table II shows the complement inhibiting activity of the intermediate compounds of the invention.

**TABLE I**

**Biological Activities**

| Compound | Cl 026* Wells | C-Late 035* Wells | Shunt Inhibition 036* Wells | Cap 50* | In Vivo Activity (Guinea Pig) % Inhibition Intraperitoneal Time (minutes) 30 | 60 | 120 |
|---|---|---|---|---|---|---|---|
| Suramin | +4** | +2** | - | 361 | -9 | -17 | -44 |
| 8,8'-[Ureylenebis[(4-methyl-3,1-phenylenecarbonyl)imino-(4-methyl-3,1-phenylenecarbonyl)-imino]]di-1,3,5-naphthalenetrisulfonic acid, hexasodium salt | +3 | +3 | N | 144 | -58 | -65 | -77 |

* Code designation for tests employed as referred herein.
**Activity in wells a serial dilution assay. Higher well number indicates higher activity. The serial dilutions are two-fold.
N=Negative (no activity)

Biological Activities

TABLE I (CONTINUED)

| Compound | Cl 026* Wells | C-Late 035* Wells | Shunt Inhibition 036* Wells | Cap 50* | In Vivo Activity (Guinea Pig) % Inhibition Intraperitoneal Time (Minutes) 30 | 60 | 120 |
|---|---|---|---|---|---|---|---|
| Suramin | +4** | +2** | - | 361 | - 9 | -17 | -44 |
| 5,5"-Ureylenebis[2'-methyl-5'-(4,6,8-trisulfo-1-naphthyl) carbamoyl)-isophthalanilic acid], octasodium salt | +5 | +2 | +2** | 249 | -17 | -50 | -57 |
| 5,5"-Ureylenebis[2'-methyl-5'-(4,6,8-trisulfo-1-naphthylcarbamoyl]-isophthalanilic acid], hexasodium salt | +5 | +2 | +2 | 295 | | | |
| 5,5'-Ureylenebis[(4-methyl-3,1-phenylenecarbonyl)imino] bis[N-(4,6,8-trisulfo-1-naphthyl)isophthalamic acid], hexasodium salt | +5 | +2 | +1 | 47 | | | |

TABLE I (continued)

| Compound | Cl 026* Wells | C-Late 035* Wells | Shunt Inhibition 036* Wells | Cap 50* | In Vivo Activity (Guinea Pig) % Inhibition Intraperitoneal Time (Minutes) 30 | 60 | 120 |
|---|---|---|---|---|---|---|---|
| 5,5'-[Ureylenebis[(4-methyl-3,1-phenylenecarbonylimino)]bis[N-(4,6,8-trisulfo-1-naphthyl)]isophthalamic acid, octasodium salt | +5 | - | - | | | | |
| 8,8'-[Ureylenebis[(2-sulfo-4,1-phenylenecarbonyl)imino-(4-methyl-3,1-phenylenecarbonylimino)]]di-1,3,5-naphthalenetrisulfonic acid, octasodium salt | +3 | +1 | +2 | 187 | | | |

* Code designation for tests employed as referred herein.
** Activity in wells a serial dilution assay. Higher well number indicates higher activity. The serial dilutions are two-fold.

TABLE I (CONTINUED)

Biological Activities

| Compound | Cl 026* Wells | C-Late 035* Wells | Shunt Inhibition 036* Wells | Cap 50* | In Vivo Activity (Guinea Pig) % Inhibition: Intraperitoneal Time (minutes) 30 | 60 | 120 | Intravenous Time (minutes) 2 | 30 | 120 |
|---|---|---|---|---|---|---|---|---|---|---|
| Suramin | +4** | +2** | | 361 | - 9 | -17 | -44 | | | |
| 8,8'-[Ureylenebis[(2-sulfo-4,1-phenylenecarbonyl)imino](2-sulfo-4,1-phenylenecarbonyl)imino]]di-1,3,6-naphthalenetrisulfonic acid, decasodium salt | +8 | +1 | +5**<br>+3 | 270 | -68 | -24 | -14 | | | |
| 8,8'-[Ureylenebis[(2-sulfo-4,1-phenylenecarbonyl)imino(2-sulfo-4,1-phenylenecarbonyl)imino]]di-1,3,5-naphthalenetrisulfonic acid, decasodium salt | +7 | +1 | +4 | 113 | -31 | -36 | -58 | -38 | -47 | -22 |
| 5,5'-[Ureylenebis[(2-sulfo-4,1-phenylenecarbonyl)imino]]bis[N-4,6,8-trisulfo-1-naphthylisophthalamic acid], octasodium salt | +6 | +2 | +5 | 83 | | | | | | |

**TABLE I (cont'd.)**

**Biological Activities**

| Compound | C1 026* Wells | C-Late 035* Wells | Shunt Inhibition 036* Wells | Cap 50* | In Vivo Activity (Guinea Pig) % Inhibition: Intraperitoneal, Time (minutes) 30 | Intraperitoneal 60 | Intraperitoneal 120 | Intravenous, Time (minutes) 2 | Intravenous 30 | Intravenous 120 |
|---|---|---|---|---|---|---|---|---|---|---|
| 5,5'-Ureylenebis[N-[3-carboxy-5-(4,6,8-trisulfo-1-naphthylcarbamoyl)phenyl]isophthalamic acid], decasodium salt | +6 | +2 | +4 | | -86 | -89 | -96 | | | |
| 5,5'-Ureylenebis[N-[3-carboxy-5-(4,6,8-trisulfo-1-naphthylcarbamoyl)phenyl]isophthalamic acid], hexasodium salt | +5 | +2 | +3 | 17 | | | | | | |

* Code designation for tests employed as referred herein.

** Activity in wells a serial diluteion assay. Higher well number indicates higher activity. The serial dilutions are two-fold.

N= Negative (no activity)

Biological Activities

TABLE I (CONTINUED)

| Compound | Cl 026* Wells | C-Late 035* Wells | Shunt Inhibition 036* Wells | Cap 50* | In Vivo Activity (Guinea Pig) % Inhibition | | | | | |
|---|---|---|---|---|---|---|---|---|---|---|
| | | | | | Intraperitoneal Time (Minutes) | | | Intravenous Time (Minutes) | | |
| | | | | | 30 | 60 | 120 | 2 | 30 | 120 |
| Suramin | +4** | +2** | - | 361 | - 9 | -17 | -44 | | | |
| 5,5"-Ureylenebis[2'-methyl-5'-[(4,6,8-trisulfo-1-naphthyl)carbamoyl]isophthalanilic acid], octasodium salt | +5 | +2 | +2** | 249 | -17 | -50 | -57 | | | |
| 5,5"-Ureylenebis[2'-methyl-5'-[(4,6,8-trisulfo-1-naphthyl)carbamoyl]isophthalanilic acid], hexasodium salt | +5 | +2 | +2 | 295 | | | | | | |
| 8,8'-[Ureylenebis[[(2-sulfo-4,1-phenylenecarbonyl)imino](2-methyl-4,1-phenylene)sulfonyl]imino]]di-1,3,6-naphthalenetrisulfonic acid, octasodium salt | +4 | +2 | +2 | 380 | | | | | | |

**Biological Activities**

**TABLE I (cont'd)**

| Compound | Cl 026* Wells | C-Late 035* Wells | Shunt Inhibition 036* Wells | Cap 50* | *In Vivo* Activity (Guinea Pig) % Inhibition | | | | | |
|---|---|---|---|---|---|---|---|---|---|---|
| | | | | | Intraperitoneal | | | Intravenous | | |
| | | | | | Time (Minutes) | | | Time (Minutes) | | |
| | | | | | 30 | 60 | 120 | 2 | 30 | 120 |
| 5,5'-Ureylenebis[(4-methyl-3,1-phenylenecarbonyl)imino]bis[N-(4,6,8-trisulfo-1-naphthyl)isophthalamic acid], hexasodium salt | +5 | +2 | +1 | 47 | | | | | | |
| 5,5'-[Ureylenebis[(4-methyl-3,1-phenylene)carbonylimino]]bis[N-(4,6,8-trisulfo-1-naphthyl)isophthalamic acid, octasodium salt | +5 | - | - | | | | | | | |
| 8,8'-[Ureylenebis[[(2-sulfo-4,1-phenylenecarbonyl)imino]-(4-methyl-3,1-phenylenecarbonyl)imino]]di-1,-3,5-naphthalenetrisulfonic acid, octasodium salt | +3 | +1 | +2 | 187 | | | | | | |

**Biological Activities**

**TABLE I (cont'd)**

| Compound | Cl 026* Wells | C-Late 035* Wells | Shunt Inhibition 036* Wells | Cap 50* | In Vivo Activity (Guinea Pig) % Inhibition — Intraperitoneal Time (Minutes) 30 | 60 | 120 | Intravenous Time (Minutes) 2 | 30 | 120 |
|---|---|---|---|---|---|---|---|---|---|---|
| 8,8'-[Ureylenebis[[(2-sulfo-4,1-phenylenecarbonyl)imino][(4,6-dimethyl-3,1-phenylenesulfonyl)imino]]-1,3,6-naphthalenetrisulfonic acid, octasodium salt | +5 | +2 | - | >330 | | | | | | |
| 8,8'-[Ureylenebis[[[[(2-sulfo-1,4-phenylenecarbonyl)imino-1,4-phenylene]sulfonyl]imino]]di-1,3,6-naphthalenetrisulfonic acid, octasodium salt | +5 | N | +1 | 250 | | | | -92 | -56 | -25 |

* Code designation for tests employed as referred herein.
** Activity in wells a serial dilution assay. Higher well number indicates higher activity. The serial dilutions are two-fold.

TABLE I (CONTINUED)

Biological Activities

| Compound | Cl 026* Wells | C-Late 035* Wells | Shunt Inhibition 036* Wells | Cap 50* |
|---|---|---|---|---|
| Suramin | +4** | +2** | - | 361 |
| 8,8'-[Ureylenebis[(m-phenylenesulfonylimino)(4-methyl-3,1-phenylenesulfonyl)imino]]di-1,3,5-naphthalenetrisulfonic acid, hexasodium salt | +3 | +1 | N | >500 |
| 8,8'-[Ureylenebis[[(1,3-phenylenesulfonylimino)-1,3-phenylenesulfonyl]imino]]di-1,3,6-naphthalenetrisulfonic acid, hexasodium salt | +4 | +5 | +1** | >500 |
| 8,8'-[Ureylenebis[[[(6-methyl-3,1-phenylene)sulfonyl]imino]-[[(6-methyl-3,1-phenylene)sulfonyl]imino]]]di-1,3,6-naphthalenetrisulfonic acid, hexasodium salt | +3 | +1 | N | >500 |

* Code designation for tests employed as referred herein.
**Activity in wells a serial dilution assay. Higher well number indicates higher activity. The serial dilutions are two-fold.
N=Negative (no activity)

TABLE II (INTERMEDIATES)

Biological Activities

| Compound | Cl 026* Wells | C-Late 035* Wells | Shunt Inhibition 036* Wells | Cap 50* |
|---|---|---|---|---|
| 5-Amino-2'-methyl-5'(4,6,8-tri-sulfo-1-naphthyl)carbamoyl)iso-phthalanilic acid, trisodium salt | +2** | N | N | |
| 5-(3-Amino-p-toluamido)-N-(4,6,8-trisulfo-1-naphthyl)isophthalamic acid, trisodium salt | +3 | N | N | >500 |
| 8-[3-(4-Amino-2-sulfobenzamido)-p-toluamido]-1,3,5-naphthalenetrisulfonic acid, tetrasodium salt | +2 | N | N | |

* Code designation for tests employed as referred herein.
** Activity in wells a serial dilution assay. Higher well number indicates higher activity. The serial dilutions are two-fold.
N= Negative (no activity).

TABLE II (Intermediates) (CONTINUED)

Biological Activites

| Compound | Cl 026* Wells | C-Late 035* Wells | Shunt Inhibition 036* Wells | Cap 50* | In Vivo Activity (Guinea Pig) % Inhibition | | | | | |
|---|---|---|---|---|---|---|---|---|---|---|
| | | | | | Intraperitoneal Time (minutes) | | | Intravenous Time (minutes) | | |
| | | | | | 30 | 60 | 120 | 2 | 30 | 120 |
| 8-[4-(4-Amino-2-sulfobenzamido)-2-sulfobenzamido]-1,3,6-naphthalenetrisulfonic acid, pentasodium salt | +5** | N | N | 420 | | | | -77 | -43 | -31 |
| 8-[4-(4-Amino-2-sulfobenzamido)-2-sulfobenzamido]-1,3,5-naphthalenetrisulfonic acid, pentasodium salt | +4 | N | N | 177 | -15 | -17 | -23 | -52 | -28 | -10 |
| 5-(4-Amino-2-sulfobenzamido)-N-(4,6,8-trisulfo-1-naphthyl)isophthalamic acid, tetrasodium salt | +3 | N | N | 242 | | | | | | |

TABLE II (Intermediates) (cont'd.)

Biological Activities

| Compound | Cl 026* Wells | C-Late 035* Wells | Shunt Inhibition 036* Wells | Cap 50* | In Vivo Activity (Guinea Pig) % Inhibition | | | | | |
|---|---|---|---|---|---|---|---|---|---|---|
| | | | | | Intraperitoneal Time (minutes) | | | Intravenous Time (minutes) | | |
| | | | | | 30 | 60 | 120 | 2 | 30 | 120 |
| 5-Amino-3'-carboxy-5'-(4,6,8-trisulfo-1-naphthylcarbamoyl) isophthalanilic acid, trisodium salt | +3 | N | N | 107 | | | | | | |

* Code designation for tests employed as referred herein.

** Activity in wells a serial dilution assay. Higher well number indicates higher activity. The serial dilutions are two-fold.

N= Negative (no activity).

TABLE II (INTERMEDIATES) (CONTINUED)

Biological Activities

| Compound | Cl 026* Wells | C-Late 035* Wells | Shunt Inhibition 036* Wells | Cap 50* |
|---|---|---|---|---|
| 5-Amino-2'-methyl-5'-[(4,6,8-trisulfo-1-naphthyl)carbamoyl]isophthalanilic acid, trisodium salt | +2** | N | N | |
| 8-[5-(4-Amino-2-sulfobenzamido)-o-toluenesulfonamido]-1,3,6-naphthalenetrisulfonic acid, tetrasodium salt | +4 | N | N | >500 |
| 5-(3-Amino-p-toluamido)-N-(4,6,8-trisulfo-1-naphthyl)isophthalamic acid, trisodium salt | +3 | N | N | >500 |
| 8-[3-(4-Amino-2-sulfobenzamido)-p-toluamido]-1,3,5-naphthalenetrisulfonic acid, tetrasodium salt | +2 | N | N | |

TABLE II (INTERMEDIATES) (cont'd)

Biological Activities

| Compound | Cl 026* Wells | C-Late 035* Wells | Shunt Inhibition 036* Wells | Cap 50* |
|---|---|---|---|---|
| 5-Amino-2'-methyl-5'-(4,6,8-trisulfo-1-naphthylsulfamoyl)isophthalanilic acid, trisodium salt | +2 | N | N | |
| 8-[$\underline{N}^3$-(4-Amino-2-sulfobenzoyl)-metanilamido]-1,3,6-naphthalenetrisulfonic acid, tetrasodium salt | +3 | N | N | |

* Code designation for tests employed as referred herein.
** Activity in wells a serial dilution assay. Higher well number indicates higher activity. The serial dilutions are two-fold.
N= Negative (no activity).

The following examples will serve to illustrate the invention in more detail.

EXAMPLE 1

8-[m-(m-Aminobenzamido)benzamido]-1,3,6-naphthalenetrisulfonic acid, trisodium salt

A 53.9 g portion of 8-amino-1,3,6-naphthalenetrisulfonic acid, trisodium salt (Eastman) is combined with 120 ml of 1N sodium hydroxide, 60 ml of water and 45.0 g of m-nitrobenzoyl chloride. The reaction mixture sets up to an unshakable sludge. A 120 ml portion of 1N sodium hydroxide and 60 ml of water are added and the mixture is shaken for 10-15 minutes. At intervals, two more 120 ml portions of 1N sodium hydroxide are added and the mixture is shaken 45 minutes after the addition of the last portion. The mixture is acidified with 15 ml of concentrated HCl and extracted copiously with ehter. The aqueous phase is filtered through sintered glass and the filtrate is neutralized. The filtrate is concentrated in vacuo at 55°-60°C to 1/2 volume, forming a solid. A 200 ml portion of saturated saline and 100 ml of water are added and the mixture is triturated, filtered and washed with 200 ml of saturated saline, two 300 ml portions of 90% ethanol, 400 ml of absolute alcohol and two 300 ml portions of ether giving 71.8 g of 8-m-nitrobenzamido-1,3,6-naphthalenetrisulfonic acid, trisodium salt.

A 50.0 g portion of the product above, in 210 ml of water containing 4.0 g of 10% palladium on carbon catalyst, is hydrogenated at room temperature and an initial pressure of 42 pounds for 4 3/4 hours. The mixture is filtered. The filtrate is concentrated in vacuo at 55°-60°C to a low volume, diluted with absolute ethanol, filtered and washed with absolute ethanol, giving 43.06 g of 8-(m-aminobenzamido)-1,3,6-naphthalenetrisulfonic acid, trisodium salt.

A 25.0 g portion of the preceding product, 45 ml of 1N NaOH, 30 ml of water and 16.5 g of m-nitrobenzoyl chloride are combined and shaken. Three additional 45 ml portions of 1N NaOH are added with shaking after each portion.

The mixture is acidified with 10 ml of concentrated HCl and extracted with seven 150 ml portions of ether. The ether extracts are syphoned off and 200 ml of water is added to solubilize the product. The aqueous phase is neutralized and concentrated in vacuo at 50°-55°C until a solid precipitates. After standing overnight at room temperature, the mixture is filtered and the solid is washed with 200 ml of saturated saline. The moist paste is dissolved in 50 ml of hot water and diluted with 250 ml of hot absolute alcohol, resulting in a thick paste. The paste is diluted with 100 ml of 80% ethanol and filtered. The solid is slurried on the funnel with 250 ml of absolute alcohol and then filtered giving 25.48 g of 8-[m-(m-nitrobenzamido)benzamido]-1,3,6-naphthalenetrisulfonic acid, trisodium salt.

A 20.0 g portion of the product above, in 160 ml of water containing 2.0 g of 10% palladium on carbon catalyst is hydrogenated at room temperature and 44 pounds pressure for 3 hours. The catalyst is filtered off and the filtrate is evaporated at 50°-55°C. The resulting oil is diluted with absolute alcohol, triturated, filtered and washed with absolute alcohol giving 17.84 g of the product of the Example.

EXAMPLE 2

8-[3-(3-Amino-p-toluamido)-p-toluamido]-1,3,5-naphthalenetrisulfonic acid, trisodium salt

A mixture of 25.0 g of 4-methyl-3-nitrobenzoic acid and 50 ml of thionyl chloride is refluxed for 3 1/2 hours in a 110°C oil bath. The resulting solution is evaporated in vacuo to an oil. The oil is distilled at a pressure of 0.5 mm of mercury and an oil bath temperature of 145°-160°C. The fraction, bp 108°-113°C, is collected to give 24.3 g of 3-nitro-p-toluoyl chloride.

To a stirred solution of 22.5 g of 8-amino-1,3,5-naphthalenetrisulfonic acid, trisodium salt in 160 ml of water is added 11.0 g of the preceding product with a small amount of ether. Stirring is continued, and after one hour 1.0 g of sodium acetate trihydrate and 1.0 g of 3-nitro-p--toluoyl chloride are added. The mixture is stirred an additional 3 hours and the above addition of sodium acetate and 3-nitro-p-toluoyl chloride is repeated. The mixture is stirred an additional hour, acidified to Congo Red indicator paper with hydrochloric acid and filtered. The filtrate is neutralized with sodium hydroxide, concentrated, dissolved in 50 ml of water and added to one liter of ethanol with sitrring for 16 hours. The solid is filtered and forms a gel on washing with ethanol. The gel is dissolved in water and evaporated. The residue is dissolved in 35 ml of hot water and 320 ml of absolute ethanol is added with stirring. The mixture solidifies and water is added to allow filtration. The solid is washed with ether and dried in vacuo. The filtrate is treated by stirring with one liter of ethanol, the separated solid is collected, washed with ether, and dried to yield a combined total of 23.9 g of 8-(3-nitro-p-toluamido)-1,3,5-naphthalenetrisulfonic acid, trisodium salt.

A 22.0 g portion of the preceding product, 200 ml of water and 2.5 g of 10% palladium on carbon catalyst is hydrogenated in a Parr shaker until no additional hydrogen is absorbed. The resulting mixture is filtered through diatomaceous earth, the residue is washed with water, and the filtrate is evaporated, the residue is dissolved in 50 ml of water and added to 900 ml of absolute ethanol. The mixture is warmed on a steam bath and then is stirred at room temperature for 3 hours. The mixture is filtered and the solid is washed with ethanol, then ether and dried in vacuo to give 16.89 g of 8-(3-amino-p-toluamido)-1,3,5-naphthalenetrisulfonic acid, trisodium salt.

To a stirred solution of 3.7 g of the above product, 25.0 ml of water and 670 mg of anhydrous sodium carbonate is added 1.56 g of 3-nitro-p-toluoyl chloride. The mixture is stirred at room temperature for 16 hours. The reaction mixture is filtered, the filtrate is acidified with glacial acetic acid and is evaporated. The residue is dissolved in 20 ml of hot water and ethanol is added. The mixture is heated on a steam bath until solution is achieved then 160 ml

of ethanol is added and heating is continued again until solution is complete. The solution is allowed to cool and forms a gelatinous precipitate. The product is collected and is washed with ethanol and ether to yield 4.3 g of 8-[3-(3--nitro-p-toluamido)-p-toluamido]-1,3,5-naphthalenetrisulfonic acid, trisodium salt.

A mixture of 3.0 g of the preceding compound, 125 ml of water and 400 mg of palladium on carbon catalyst is hydrogenated until no additional hydrogen is absorbed. The reaction mixture is filtered through diatomaceous earth and the filtrate is evaporated. The residue is dissolved in hot water and ethanol is added to separate a small amount of black oil. The solution is decanted and ethanol is added to it separating a tan oil. The procedure is repeated several times until no additional oil separates, then the separated oils (excluding the first) are combined and dissolved in 25.0 ml of hot water. This solution is added to 225 ml of anhydrous ethanol, with vigorous stirring, to form a white precipitate. The product is collected and is washed with ethanol and ether to give 2.88 g of the product of the Ecample as a white powder.

EXAMPLE 3

8,8'-[Ureylenebis[(4-methyl-3,1-phenylenecarbonyl)-imino(4-methyl-3,1-phenylenecarbonyl)imino]di--1,3,5-naphthalenetrisulfonic acid, hexasodium salt

Phosgene is bubbled into a vigorously stirred solution of 2.88 g of the product of Example 2, 25 ml of water and 440 mg of anhydrous sodium carbonate until the solution is acidic to Congo Red indicator paper. The mixture is neutralized with sodium carbonate, then an additional 440 mg of sodium carbonate is added and phosgenation is continued until the mixture is again acidic. The mixture is made alkaline to pH 8.2 with sodium carbonate, then is filtered. The filtrate is evaporated and the residue is dissolved in 20.0 ml of hot water. The slow addition of 80.0 ml of ethanol provides an oil. The mixture is evapora-porated and the residue is treated as above with 20 ml of hot water and 80 ml of ethanol to yield an oil, then 10 ml of water and 40 ml of ethanol is added and the solution is stirred for 16 hours. A white precipitate suspended in solution is collected by filtration by decanting from a brown gum. The precipitate is washed with ethanol and ether to yield a white powder. Additional white powder is recovered from the above filtrate. The gum is dissolved in water and concentrated, then is heated with 80% aqueous ethanol and is filtered. The ethanol extraction is repeated 3 times, the extracts are combined and evaporated to yield a white solid. The products above are combined and dissolved in 20 ml of hot water. The aqueous solution is added to 180 ml of absolute ethanol with vigorous stirring to provide a precipitate. The precipitate is collected and is washed with ethanol and ether. The product is reprecipitated from water and ethanol, then is collected and washed as above and dried to yield 1.62 g of the product of the Example as a white powder.

EXAMPLE 4

8-[3-(3-Amino-p-toluenesulfonamido)-p-toluamido]-
-1,3,5-naphthalenetrisulfonic acid, trisodium salt

Following the procedure of Example 2, reaction of 3-nitro-p-toluenesulfonyl chloride with 8-(3-amino-p-toluamido)-1,3,5-naphthalenetrisulfonic acid, trisodium salt, followed by reduction with palladium on carbon catalyst provides the product of the Example.

EXAMPLE 5

8,8'-[Ureylenebis[(4-methyl-3,1-phenylenesulfonyl)-
imino(4-methyl-3,1-phenylenecarbonyl)imino]]di-
-1,3,5-naphthalenetrisulfonic acid, hexasodium salt

Following the procedure of Example 3, reaction of the product of Example 4 with phosgene provides the product of the Example.

EXAMPLE 6

8-[3-(5-Amino-o-toluenesulfonamido)-p-toluamido]--1,3,5-naphthalenetrisulfonic acid, trisodium salt

Following the procedure of Example 2, reaction of 5-nitro-o-toluenesulfonylchloride with 8-(3-amino-p-toluamido)-1,3,5-naphthalenetrisulfonic acid, trisodium salt, followed by reduction with palladium on carbon catalyst provides the product of the Example.

EXAMPLE 7

8,8'-[Ureylenebis[(6-methyl-3,1-phenylenesulfonyl)-imino(4-methyl-3,1-phenylenecarbonyl)imono]]di--1,3,5-naphthalenetrisulfonic acid, hexasodium salt

Following the procedure of Example 3, reaction of the product of Example 6 with phosgene provides the product of the Example.

EXAMPLE 8

8-[3-(5-Amino-2,4-xylenesulfonamido)-p-toluamido]--1,3,5-naphthalenetrisulfonic acid, trisodium salt

Following the procedure of Example 2, reaction of 5-nitro-2,4-xylenesulfonyl chloride (from 2-nitro-2,4-xylenesulfonic acid and thionyl chloride) with 8-(3-amino-p-toluamido)-1,3,5-naphthalenetrisulfonic acid, trisodium salt, followed by reduction with palladium on carbon catalyst provides the product of the Example.

EXAMPLE 9

8,8'-[Ureylenebis[(4,6-dimethyl-3,1-phenylenesulfonyl)imino(4-methyl-3,1-phenylenecarbonyl)imino]]-di-1,3,5-naphthalenetrisulfonic acid, hexasodium salt

Following the procedure of Example 3, reaction of the product of Example 8 with phosgene provides the product of the Example.

EXAMPLE 10

8-[3-(3-Amino-p-toluenesulfonamido)benzamido]--1,3,5-naphthalenetrisulfonic acid, trisodium salt

Following the procedure of Example 2, reaction of

3-nitrobenzoyl chloride with 8-amino-1,3,5-naphthalenetrisulfonic acid, trisodium salt provides 8-(3-nitrobenzamido)-1,3,5-naphthalenetrisulfonic acid, trisodium salt. Reduction of the latter in water with 10% palladium on carbon catalyst gives the corresponding 3-amino compound. Reaction of this intermediate with 3-nitro-p-toluenesulfonyl chloride followed by reduction with palladium on carbon catalyst provides the product of the Example.

EXAMPLE 11

8,8'-[Ureylenebis[(4-methyl-3,1-phenylenesulfonyl)imino-3,1-phenylenecarbonylimino]]-di-1,3,5-naphthalenetrisulfonic acid, hexasodium salt

Following the procedure of Example 3, reaction of the product of Example 10 with phosgene provides the product of the Example.

EXAMPLE 12

5-Amino-2'-methyl-5'-(4,5,8-trisulfo-1-naphthylcarbamoyl) isophthalanilic acid, trisodium salt

To a warm solution of 106.4 g of (80.5%) 8-amino-1,3,5-naphthalenetrisulfonic acid in 100 ml of water and 45.0 ml of 5N sodium hydroxide is slowly added 500 ml of absolute ethanol with vigorous stirring for 30 minutes. The mixture is cooled to room temperature and filtered. The precipitate is washed with 80% aqueous ethanol, ethanol and ether and is dried *in vacuo* at 110°C for 16 hours to give 103.7 g of 8-amino-1,3,5-naphthalenetrisulfonic acid trisodium salt.

A mixture of 25.0 g of 4-methyl-3-nitrobenzoic acid and 50 ml of thionyl chloride is refluxed for 3 1/2 hours in a 110°C oil bath. The resulting solution is evaporated *in vacuo* to an oil. The oil is distilled at a pressure of 0.5 mm of mercury and an oil bath temperature of 145°-160°C. The fraction, bp 108°-113°C, is collected to give 24.3 of 3-nitro-p-toluoyl chloride.

To a stirred solution of 22.5 g of 8-amino-1,3,5-naphthalenetrisulfonic acid trisodium salt in 160 ml of

water is added 11.0 g of the preceding product with a small amount of ether. Sti-ring is continued, and after one hour 1.0 g of sodium acetate trihydrate and 1.0 g of 3-nitro-p--toluoyl chloride are added. The mixture is stirred an additional 3 hours and the above addition of sodium acetate and 3-nitro-p-toluoyl chloride is repeated. The mixture is stirred an additional hour, acidified to Congo red indicator paper with hydrochloric acid and filtered. The filtrate is neutralized with sodium hydroxide, concentrated, dissolved in 50 ml of water and added to one liter of ethanol with stirring for 16 hours. The solid is filtered and forms a gel on washing with ethanol. The gel is dissolved in water and evaporated. The residue is dissolved in 35 ml of hot water and 320 ml of absolute ethanol is added with stirring. The mixture solidifies and water is added to allow filtration. The solid is washed with ether and dried in vacuo. The filtrate is treated by stirring with one liter of ethanol, the separated solid is collected, washed with ether, and dried to yield a combined total of 23.9 g of 8-(3-nitro-p-toluamido)-1,3,5-naphthalenetrisulfonic acid trisodium salt.

A 22.0 g portion of the preceding product, 200 ml of water and 2.5 g of 10% palladium-on-carbon catalyst is hydrogenated in a Parr shaker until no additional hydrogen is absorbed. The resulting mixture is filtered through diatomaceous earth, the residue is washed with water, and the filtrate is evaporated. The residue is dissolved in 50 ml of water and added to 900 ml of absolute ethanol. The mixture is warmed on a steam bath, then is stirred at room temperature for 3 hours. The mixture is filtered and the solid is washed with ethanol, then ether and dried in vacuo to give 16.89 g of 8-(3-amino-p-toluamido)-1,3,5-naphthalenetrisulfonic acid trisodium salt.

A mixture of 60.0 g of 5-nitroisophthalic acid, 300 ml of thionyl chloride and one ml of dimethylformamide is stirred at room temperature for 30 minutes, then is refluxed for one hour. The resulting clear solution is allowed

to stand 24 hours, then is evaporated to a small volume *in vacuo*. The evaporation step is repeated with toluene and the resulting liquid is diluted with 250 ml of hexane. The mixture is stirred and cooled until the resulting oil is solidified. The product is ground to a powder and is recrystallized twice from carbon tetrachloride to give 47.4 g of 5-nitroisophthaloyl chloride.

A solution of 2.78 g of 5-nitroisophthaloyl chloride in 50 ml of ether is added to a stirred mixture of 6.5 g of 8-(3-amino-*p*-toluamido)-1,3,5-naphthalenetrisulfonic acid trisodium salt, 3.2 g of sodium acetate trihydrate, 50 ml of water and 40 ml of ether over a 40 minute period. The mixture is stirred for an additional 30 minutes, then is evaporated. The residue is dissolved in 20 ml of hot water, then is placed in an icebox overnight. The precipitate is collected, washed with ice-water, 80% aqueous ethanol, ethanol and ether and is dried to yield 2.9 g of 2'-methyl-5-nitro--5'-(4,6,8-trisulfo-1-naphthylcarbamoyl)isophthalanilic acid, trisodium salt.

A mixture of 4.21 g of the product prepared as described above, 120 ml of water and 0.70 g of 10% palladium-on-carbon catalyst is hydrogenated on a Parr shaker until no additional hydrogen is absorbed. The reaction mixture is filtered through diatomaceous earth and the filtrate is evaporated. The residue is dissolved in 20 ml of hot water and ethanol is added to a total volume of 250 ml with formation of a gel. The gel is separated, washed with ethanol and ether and dried to yield 3.25 g of the product of the Example.

## EXAMPLE 13

**5,5"-Ureylenebis[2'-methyl-5'-(4,6,8-trisulfo-1-naphthylcarbamoyl)isophthalanilic acid], octasodium salt**

A solution of 3.0 g of the product of Example 12 and 0.90 g of anhydrous sodium carbonate in 30 ml of water is phosgenated until it is acidic to Congo Red indicator paper. The pH of the solution is adjusted to 7.0 with anhydrous sodium carbonate, the solution is filtered, and the

filtrate is evaporated. The residue is dissolved in 20 ml of hot water, then 80 ml of absolute ethanol is added with formation of a gum. The supernatant is decanted and set aside for Example 3. The gum is triturated with ethanol to provide a solid. The solid is stirred for 2 hours in ethanol and is collected. The solid is washed with ethanol and ether and is dried to yield 2.0 g of the product of the Example.

EXAMPLE 14

5,5"-Ureylenebis[2'-methyl-5'-(4,6,8-trisulto-1-naphthylcarbamoyl)isophthalanilic acid], hexasodium salt

The decanted supernatant set aside in Example 13 separates a solid on standing. The solid is collected and washed with ethanol and ether. The solid is dissolved in 2.0 ml of water and is acidified with hydrochloric acid, then 8.0 ml of absolute ethanol is added with formation of a precipitate. The product is collected, washed with ethanol and ether and dried to give 175 mg of the product of the Example.

EXAMPLE 15

5-(3-Amino-p-toluamido)-N-(4,6,8-trisulfo-1-naphthyl)isophthalamic acid, trisodium salt

A 35.0 g portion of 5-nitroisophthaloyl chloride is added to 600 ml of methanol with stirring producing a precipitate. The mixture is heated to solution, then is chilled, filtered and dried to yield 31.75 g of dimethyl 5-nitroisophthalate.

A mixture of 7.46 g of potassium hydroxide in 87.5 ml of methanol is added to a stirred solution of 31.75 g of the preceding product in 331.0 ml of acetone. A solid is precipitated and stirring is continued for 16 hours. The solid (A) is filtered off, washed with ether and set aside. The filtrate is evaporated, the residue is extracted with 125 ml of warm water and is filtered. The filtrate is acedified with dilute hydrochloric acid to produce a precipitate which is collected and dried to yield 3.4 g of product. The solid (A) above is extracted with 250 ml

of warm water and is filtered. The filtrate is filtered again at room temperature, acidified with dilute hydrochloric acid and cooled. The precipitate is collected and dried to give 18.25 g of additional product identified as 5-nitro--isophthalic acid, 3-methyl ester.

A mixture of 18.38 g of the above product, 60 ml of thionyl chloride and 0.37 ml of dimethylformamide is heated at 60°C for 2.5 hours. The solution is evaporated, then is treated with toluene, and again is evaporated. The residue is slurried in hot diethyl ether and the ether volume is reduced by evaporation. The mixture is chilled and filtered. The precipitate is washed with cold ether and is dried. The material is extracted with 500 ml of boiling hexane by decantation. The hexane is cooled and filtered to yield 14.1 g of 3-carbomethoxy-5-nitrobenzoyl chloride.

To a solution of 14.0 g of 8-amino-1,3,5-naphthalenetrisulfonic acid trisodium salt and 8.96 g of sodium acetate trihydrate in 150 ml of water is added with stirring 8.0 g of 3-carbomethoxy-5-nitrobenzoyl chloride. Stirring is continued for 2 hours then 18.0 ml of diethyl ether is added and stirring is continued for one additional hour. An additional 1.12 g of sodium acetate is added along with 1.0 g of 3-carbomethoxy-5-nitrobenzoyl chloride with continued stirring for one hour. The mixture is filtered and the filtrate is concentrated. The residue is dissolved in 100 ml of hot water, then 100 ml of absolute ethanol is added with formation, on standing, of a precipitate. The material is filtered, washed with 80% aqueous ethanol, ethanol and ether and dried to yield 17.35 g of 5-nitro-N-4,6,8-trisulfo-1--naphthylisophthalamic acid methyl ester, trisodium salt.

A 3.27 g portion of the preceding product and 700 mg of 10% palladium-on-carbon catalyst in 100 ml of water is hydrogenated in a Parr shaker until no more hydrogen is absorbed. The resulting mixture is filtered through diatomaceous earth and the filtrate is concentrated. The residue is dissolved in about 15 ml of hot water and absolute ethanol is added to a total volume of 250 ml with formation of a

precipitate. The precipitate is collected by filtration, washed with ethanol and ether and dried to yield 2.4 g of 5-amino-N-4,6,8-trisulfo-1-naphthylisophthalamic acid methyl ester, trisodium salt.

A mixture of 25.0 g of 4-methyl-3-nitrobenzoic acid and 50 ml of thionyl chloride is refluxed for 3.5 hours in a 110°C oil bath. The resulting solution is evaporated in vacuo to an oil. The oil is distilled at a pressure of 0.5 mm of mercury and an oil bath temperature of 145°-160°C. The fraction, bp 108°-113°C, is collected to give 24.3 g of 3-nitro-p-toluoyl chloride.

To a stirred solution of 1.6 g of 5-amino-N-4,6,8--trisulfo-1-naphthylisophthalamic acid methyl ester, trisodium salt, 15.0 ml of water and 270 mg of sodium carbonate is added 611 mg of the preceding compound. The reaction mixture is stirred at room temperature for 3 hours, then is acidified with dilute hydrochloric acid and poured into absolute ethanol. The resulting precipitate is collected, washed with ethanol and ether and dried to yield 1.59 g of 5-(3--nitro-p-toluamido)-N-(4,6,8-trisulfo-1-naphthyl)isophthalamic acid methyl ester, trisodium salt.

A 1.39 g portion of the product above is dissolved by stirring in 50 ml of 1N sodium hydroxide. Stirring is continued for 3.5 hours, then the solution is acidified with dilute hydrochloric acid affording a white gelatinous material. Ethanol is then added and the mixture is evaporated. The residue is dissolved in hot water, is triturated with ethanol and cooled. The precipitate is collected and washed twice with both ethanol and ether and is dried to yield 460 mg of 5-(3-nitro-p-toluamido)-N-(4,6,8-trisulfo-1--naphthyl)isophthalamic acid, trisodium salt.

A mixture of 300 mg of the product above, 50.0 ml of water and 30.0 mg of 10% palladium-on-carbon catalyst is hydrogenated at room temperature for 3 hours. The resulting mixture is filtered through diatomaceous earth and the filtrate is evaporated. The residue is re-precipitated from water and ethanol and is collected and dried to yield 255 mg

of the product of the Example.

EXAMPLE 16

5,5'-[Urevlenebis[(4-methyl-3,1-phenylenecarbonyl)imino]]-bis[N-(4,6,8-trisulfo-1-naphthyl)isophthalamic acid], octasodium salt

A solution of 240 mg of the product of Example 15, 10 ml of water and 27 mg of anhydrous sodium carbonate is phosgenated until acidic to Congo Red indicator paper, then an additional 77 mg of sodium carbonate is added and phosgene is bubbled in until acidic again. The solution is neutralized with sodium carbonate and the reaction mixture is evaporated, the residue is dissolved in hot water and ethanol is added until a precipitate forms. The mixture is cooled and the precipitate is collected. The solid is washed twice with both ethanol and ether and dried to yield 199 mg of the product of the Example.

EXAMPLE 17

5,5'-Ureylenebis[(4-methyl-3,1-phenylenecarbonyl)-imino]bis[N-(4,6,8-trisulfo-naphthyl)isophthalamic acid], hexasodium salt

A solution of 100 mg of the product of Example 16 in 10 ml of water is acidified with glacial acetic acid. The mixture is evaporated and the residue is dissolved in hot water. Ethanol is added until a precipitate forms. The mixture is cooled and the precipitate is collected. The solid is washed twice with both ethanol and ether and is dried to yield 85.0 mg of the product of the Example.

EXAMPLE 18

8-[3-(4-Amino-2-sulfobenzamido)-p-toluamido]-1,3,5-naphthalenetrisulfonic acid tetrasodium salt

To a stirred solution of 6.5 g of 8-(3-amino-p--toluamido)-1,3,5-naphthalenetrisulfonic acid, trisodium salt (prepared in Example 1) and 2.4 g of sodium acetate trihydrate in 43.0 ml of water is added 3.07 g of 4-nitro-2-sulfobenzoic acid anhydride. The mixture is stirred for 30 minutes then

is filtered. The filtrate is acidified with 0.98 ml of concentrated hydrochloric acid and evaporated in vacuo. The residue is dissolved in 20 ml of hot water which is added to 130 ml of absolute ethanol forming a gelatinous precipitate. The material is collected and washed with 80% aqueous ethanol, ethanol and ether and is dried to yield 4.2 g of 8-[3-(4--nitro-2-sulfobenzamido)-p-toluamido]-1,3,5-naphthalenetrisulfonic acid, tetrasodium salt.

A mixture of 5.95 g of the preceding product, 100 ml of water and 0.80 g of 10% palladium-on-carbon catalyst is hydrogenated as described in Example 1. The resulting mixture is filtered through diatomaceous earth and the filtrate is evaporated. The residue is dissolved in 20 ml of hot water and added to 230 ml of absolute ethanol separating an oil. The supernatant is decanted and evaporated. The residue is dissolved in 10 ml of water and 230 ml of absolute ethanol is added forming a fine precipitate. The precipitate is collected, washed with ethanol and ether and dried to yield 2.95 g of the product of the Example.

## EXAMPLE 19

### 8,8'-[Ureylenebis[(2-sulfo-4,1-phenylenecarbonyl)imino(4--methyl-3,1-phenylenecarbonyl)imino]]-di-1,3,5-naphthalenetrisulfonic acid, octasodium salt

A cooled solution of 4.0 g of the product of Example 18 and 1.07 g of anhydrous sodium carbonate in 30.0 ml of water is phosgenated until acidic to Congo Red indicator paper. The pH of the solution is adjusted to 8.0 with sodium carbonate, then to pH 6.5 with glacial acetic acid. The solution is filtered and the filtrate is evaporated. The residue is dissolved in 25.0 ml of hot water, then 50 ml of absolute ethanol is added with stirring, resulting in precipitation of a product. The material is collected by filtration, washed with 80% aqueous ethanol, ethanol and ether and is dried to yield 3.0 g of the product of the Example.

## EXAMPLE 20

### 5-Amino-2'-methyl-5'-(3,6,8-trisulfo-1-naphthylcarbamoyl)isophthalanilic acid, trisodium salt

Following the procedure of Example 12 employing 8-amino-1,3,6-naphthalenetrisulfonic acid, trisodium salt provides the product of the Example.

EXAMPLE 21

5,5"-Ureylenebis[2'-methyl-5'-(3,6,8-trisulfo-1-naphthylcarbamoyl)isophthalanilic acid], octasodium salt

Following the procedure of Example 13, phosgenation of the product of Example 20 provides the product of the Example.

EXAMPLE 22

5-(3-Amino-p-toluamido)-N-(3,6,8-trisulfo-1-naphthyl)isophthalamic acid, trisodium salt

Following the procedure of Example 15 employing 8-amino-1,3,6-naphthalenetrisulfonic acid, trisodium salt provides the product of the Example.

EXAMPLE 23

5,5'-[Ureylenebis[(4-methyl-3,1-phenylenecarbonyl)imino]]-bis[N-(3,6,8-trisulfo-1-naphthyl)isophthalamic acid], octasodium salt

Following the procedure of Example 16, phosgenation of the product of Example 22 provides the ureylene of the Example.

EXAMPLE 24

8-[3-(4-Amino-2-sulfobenzamido)-p-toluamido]-1,3,6-naphthalenetrisulfonic acid, tetrasodium salt

Following the procedure of Example 18, employing 8-(3-amino-p-toluamido)-1,3,6-naphthalenetrisulfonic acid, trisodium salt gives the product of the Example.

EXAMPLE 25

8,8'-[Ureylenebis[(2-sulfo-4,1-phenylenecarbonyl)imino(4-methyl-3,1-phenylenecarbonyl)imino]]-di-1,3,6-naphthalenetrisulfonic acid, octasodium salt

Following the procedure of Example 19, phosgenation of the product of Example 24 provides the ureylene of the Example.

EXAMPLE 26

8-[4-(4-Amino-2-sulfobenzamido)-2-sulfobenzamido]-1,3,6-naphthalenetrisulfonic acid pentasodium salt

A solution of 100 g of 5-nitro-o-toluenesulfonic acid in 600 ml of water plus 80 ml of 5N sodium hydroxide is heated to 90°C in a 2 liter Erlenmeyer flask, then 240 g of potassium permanganate is added portionwise to maintain reflux over one hour and 15 minutes. The mixture is filtered and the residue is washed with water. The combined filtrate and washings are concentrated in vacuo and allowed to crystallize to give 86.2 g of crude 4-nitro-2-sulfobenzoic acid, sodium potassium salt. Recrystallization from water gives 71.3 g of purified product.

The total product above is dissolved in 250 ml of water plus 35 ml of concentrated hydrochloric acid by warming on a steam bath. The solution is then diluted with 300 ml of ethanol and allowed to crystallize at room temperature. The mixture is allowed to stand 48 hours in a chill room, then is filtered. The precipitate is washed with cold 50% aqueous ethanol, then with ethanol and ether. The material is recrystallized from 200 ml of water and is dried at 110°C to give 52.0 g of 4-nitro-2-sulfobenzoic acid-2-sodium salt.

A 50.0 g portion of the preceding compound and 500 g of thionyl chloride is stirred and refluxed for 19 hours. The mixture is evaporated to dryness in vacuo and the residue is warmed with 300 ml of toluene and is filtered. The filtrate is concentrated in vacuo and the product is crystallized twice from toluene to give 30.4 g of 4-nitro-2-sulfobenzoic acid anhydride.

To an ice bath cooled solution (5°C) of 16.0 g of 8-amino-1,3,6-naphthalenetrisulfonic acid trisodium salt and 6.7 g of sodium acetate trihydrate in 100 ml of water is added 8.8 g of 4-nitro-2-sulfobenzoic acid anhydride. The mixture is stirred vigorously for 10 minutes and is filtered. The filtrate is cooled in an ice bath and diluted with 500 ml of cold ethanol. The mixture is filtered and the product is washed with ethanol and ether and then is dried. The product is dissolved in 50 ml of warm water and is stirred

for 10 minutes after the addition of one ml of acetone. The mixture is treated with activated charcoal, filtered through diatomaceous earth and is washed with 20 ml of water. The filtrate is cooled in an ice bath and acidified with 1.5 ml of concentrated hydrochloric acid. The solution is diluted with 500 ml of cold ethanol and the precipitated material is filtered, washed with ethanol followed by ether and dried. The above purification process is repeated without acidification. The product obtained is dried overnight at 110°C to give 18.5 g of 8-(4-nitro-2-sulfobenzamido)-1,3,6-naphthalenetrisulfonic acid tetrasodium salt.

A 17.5 g portion of the preceding product and 1.5 g of palladium-on-carbon catalyst in 150 ml of water is hydrogenated for one hour at room temperature, then is filtered through diatomaceous earth. The filtrate is concentrated and the product is precipitated by the addition of absolute ethanol. The product is collected and dried overnight in an abderhalden apparatus at 110°C to give 14.5 g of 8-(4-amino-2-sulfobenzamido)-1,3,6-naphthalenetrisulfonic acid tetrasodium salt as an off-white powder.

To an ice bath cooled solution (5°C) of 17.2 g of the preceding product (prepared as described above) and 5.24 g of sodium acetate trihydrate in 100 ml of water is added, in one portion, 6.87 g of 4-nitro-2-sulfobenzoic acid anhydride. The mixture is stirred for 10 minutes, treated with activated charcoal, and filtered through diatomaceous earth. The filtrate is poured into 750 ml of absolute ethanol in a baffle flask with vigorous stirring. The mixture is filtered and the product is washed with ethanol and ether and is dried *in vacuo*. The product is dissolved in 100 ml of water, acidified with 3.0 ml of concentrated hydrochloric acid and is poured into 500 ml of ice-cold absolute ethanol in a baffle flask, with vigorous stirring. The product is collected by filtration, washed wtih ethanol and ether, and dried to yield 19.35 g of 8-[4-(4-nitro-2-sulfobenzamido)-2-sulfobenzamido]-1,3,6-naphthalenetrisulfonic acid pentasodium salt.

A 17.5 g portion of the product above and 1.0 g of 10% palladium-on-carbon catalyst in 100 ml of water is hydrogenated for one hour in a Parr shaker. The mixture is filtered through diatomaceous earth. The filtrate is concentrated to 65.0 ml and is poured into 500 ml of vigorously stirred absolute ethanol in a baffle flask, resulting in formation of a granular precipitate and a milky suspension. The suspension is decanted and set aside. The precipitate is filtered and washed with ethanol and ether to yield 16.5 g of product.

The above suspension is filtered through diatomaceous earth and the collected material is dissolved off the filter with water. The aqueous solution is concentrated to 10 ml and poured into 100 ml of vigorously stirred ethanol to precipitate 2.26 g of additional product which is collected and washed as above. The product fractions are combined and dried to give 15.3 g of the product of the Example as a pale tan powder.

EXAMPLE 27

8,8'-[Ureylenebis[(2-sulfo-4,1-phenylenecarbonyl)-imino](2-sulfo-4,1-phenylenecarbonyl)imino]]di-1,3,6-naphthalenetrisulfonic acid, decasodium salt

A 7.13 g portion of the product of Example 26 is diluted with 40 ml of water 4.0 ml of pyridine is added and phosgene is passed through for 2-3 minutes until the solution is weakly acidic. An additional 0.4 ml of pyridine is added to neutralize the solution, then the solution is poured into 350 ml of vigorously stirred ethanol. The resulting solid is separated and washed with ethanol and ether to give a pink product. The product is dissolved in 25 ml of water. The pH of the solution is adjusted to 9.0 with 2.8 ml of 5N sodium hydroxide and the volume is adjusted to 50 ml with water. The solution is treated with activated charcoal and filtered through diatomaceous earth. The filter is washed with 40 ml of water. The combined filtrate and washings are cooled in an ice bath and gradually diluted with 800 ml of cold absolute ethanol to give a milky solution containing a gum. The mixture

is stirred in an ice-bath for 3 hours. The product is separated, pulverized and dried to yield 5.0 g of the product of the Example as a tan powder.

EXAMPLE 28

8-[4-(4-Amino-2-sulfobenzamido)-2-sulfobenzamido]-1,3,5-naphthalenetrisulfonic acid, pentasodium salt

To a warm solution of 23.8 g of 80.5% 8-amino-1,3,5-naphthalenetrisulfonic acid in 25 ml of water and 25 ml of 5N sodium hydroxide is slowly added 125 ml of absolute ethanol with vigorous stirring. The mixture is cooled to room temperature, is filtered and washed with 50 ml of 80% aqueous ethanol, then ethanol and ether. The material is dried overnight at 110°C to give 21.0 g of 8-amino-1,3,5-naphthalenetrisulfonic acid, trisodium salt as a grey powder.

A 4.49 g portion of the material above and 2.14 g of sodium acetate trihydrate is dissolved in 30 ml of water. The solution is cooled to 0°C in an ice bath and 2.72 g of 4-nitro-2-sulfobenzoic acid anhydride is added all at once. After a few minutes, the ice bath is removed and the solution is stirred for a total of 20 minutes. The solution is filtered and the filtrate acidified with 0.88 ml of concentrated hydrochloric acid. The solution is concentrated and the residue is dissolved in 10 ml of water, then is added to 200 ml of ethanol and is stirred for 1/2 hour. The precipitate is collected by filtration and washed with ethanol and ether and is dried *in vacuo*. The dried material is dissolved in 10 ml of hot water and 50 ml of absolute ethanol is added, with stirring for 1/2 hour. An additional 20 ml of ethanol is added, with stirring continued for 10 minutes. The precipitate is then collected by filtration, washed with ethanol and ether and dried in an Abderhalden apparatus at 110°C overnight to yield 4.5 g of product. The product is recycled through the procedure described above using 1.07 g of sodium acetate trihydrate, 1 36 g of 4-nitro-2-sulfobenzoic acid anhydride and 0.44 ml of concentrated hydrochloric acid, respectively. After the material is dried *in vacuo* as previously described, the dried material is dissolved in 10 ml of hot

water and 70 ml of ethanol is added slowly with stirring at room temperature for one hour. The precipitate is collected by filtration and is washed with 80% aqueous ethanol, ethanol and ether, then is dried as previously described to afford 4.5 g of 8-(p-nitro-2-sulfobenzamido)-1,3,5-naphthalenetrisulfonic acid, tetrasodium salt as a yellow solid.

A 3.9 g portion of the above compound and 400 mg of 10% palladium-on-carbon catalyst in 50 ml of water is hydrogenated, filtered and evaporated as described in Example 26. The residue obtained is dissolved in 10 ml of hot water and 100 ml of absolute ethanol is added. An oily precipitate is formed which is redissolved by addition of more ethanol. The solvent is then removed *in vacuo* to give 3.7 g of 8-(4--amino-2-sulfobenzamido)-1,3,5-naphthalenetrisulfonic acid tetrasodium salt.

To a stirred solution of 6.9 g of the preceding product and 2.15 g of sodium acetate trihydrate, in 45 ml of water at room temperature, is added 2.76 g of 4-nitro-2-sulfobenzoic acid anhydride. The mixture is stirred for one hour and filtered. The filtrate is acidified with 0.88 ml of concentrated hydrochloric acid and evaporated. The residue is dissolved in 25 ml of hot water and 100 ml of absolute ethanol is added with stirring. The solid is collected and washed with 80% ethanol. The solid is slurried with absolute ethanol, filtered, washed with ether and dried to yield 7.05 g of 8-[4-(4-nitro-2-sulfobenzamido)-2-sulfobenzamido]-1,3,5-naphthalenetrisulfonic acid, pentasodium salt.

A mixture of 6.35 g of the above product, 100 ml of water and 0.9 g of 10% palladium-on-carbon catalyst is hydrogenated on a Parr shaker until no additional hydrogen is absorbed. The reaction mixture is filtered through diatomaceous earth and the filtrate is evaporated. The residue is dissolved in 25 ml of hot water and absolute ethanol is added with stirring providing a gum. The supernatant is decanted and the gum is triturated with hot ethanol to yield a solid. The solid is collected, washed with ethanol and ether and dried to yield 5.25 g of the product of the Example.

EXAMPLE 29

5-(4-Amino-2-sulfobenzamido)-N-(4,6,8-trisulfo-1-naphthyl) isophthalamic acid, tetrasodium salt

A mixture of 60.0 g of 5-nitroisophthalic acid, 300 ml of thionyl chloride and one ml of dimethylformamide is stirred at room temperature for 30 minutes, then is refluxed for one hour. The resulting clear solution is allowed to stand 24 hours, then is evaporated to a small volume *in vacuo*. The evaporation step is repeated with toluene and the resulting liquid is diluted with 250 ml of hexane. The mixture is stirred and cooled until the resulting oil is solidified. The product is ground to a powder and is recrystallized twice from carbon tetrachloride to give 47.4 g of 5-nitroisophthaloyl chloride.

A 35.0 g portion of 5-nitroisophthaloyl chloride is added to 600 ml of methanol with stirring producing a precipitate. The mixture is heated to solution, then is chilled, filtered and dried to yield 31.75 g of dimethyl 5-nitroisophthalate.

A mixture of 7.46 g of potassium hydroxide in 87.5 ml of methanol is added to a stirred solution of 31.75 g of the preceding product in 331.0 ml of acetone. A solid is precipitated and stirring is continued for 16 hours. The solid (A) is filtered off, washed with ether and set aside. The filtrate is evaporated, the residue is extracted with 125 ml of warm water and is filtered. The filtrate is acidified with dilute hydrochloric acid to product a precipitate which is collected and dried to yield 3.4 g of product. The solid (A) above is extracted with 250 ml of warm water and is filtered. The filtrate is filtered again at room temperature, acidified with dilute hydrochloric acid and cooled. The precipitate is collected and dried to give 18.25 g of additional product identified as 5-nitro-isophthalic acid, 3-methyl ester.

A mixture of 18.38 g of the product above, 60 ml of thionyl chloride and 0.37 ml of dimethylformamide is heated at 60°C for 2.5 hours. The solution is evaporated,

then is treated with toluene, and again is evaporated. The residue is slurried in hot diethyl ether and the ether volume is reduced by evaporation. The mixture is chilled and filtered. The precipitate is washed with cold ether and is dried. The material is extracted with 500 ml of boiling hexane by decantation. The hexane is cooled and filtered to yield 14.1 g of 3-carbomethoxy-5-nitrobenzoyl chloride.

To a solution of 3.5 g of 8-amino-1,3,5-naphthalenetrisulfonic acid, trisodium salt (prepared as described in Example 28) and 2.24 g of sodium acetate trihydrate in 40 ml of water is added, with stirring, 2.0 g of 3-carbomethoxy-5-nitrobenzoyl chloride. Stirring is continued for one hour, then 4.0 ml of ethyl ether is added and stirring is continued for 2 hours longer. The solution is filtered and the filtrate is concentrated. The residue is dissolved in 20 ml of hot water and on addition of 20 ml of absolute ethanol a precipitate is formed. The precipitate is mobilized with water and is filtered and washed with 80% aqueous ethanol, ethanol and ether. The filtrate is allowed to stand overnight to afford additional product which is collected and washed as above. The combined product is dried by conventional means to yield 3.3 g of 5-nitro-N-4,6,8-trisulfo-1-naphthylisophthalamic acid methyl ester, trisodium salt.

A 3.27 g portion of the preceding product and 700 mg of 10% palladium-on-carbon catalyst in 100 ml of water is hydrogenated in a Parr shaker until no more hydrogen is absorbed. The resulting mixture is filtered through diatomaceous earth and the filtrate is concentrated. The residue is dissolved in about 15 ml of hot water and absolute ethanol is added to a total volume of 250 ml with formation of a precipitate. The precipitate is collected by filtration, washed with ethanol and ether and dried to yield 2.4 g of 5-amino-N-4,6,8-trisulfo-1-naphthylisophthalamic acid methyl ester, trisodium salt as a powder.

To a stirred solution of 1.43 g of the above product, 244 mg of anhydrous sodium carbonate and 15.0 ml of water is added 642 mg of 4-nitro-2-sulfobenzoic acid anhydride,

stirring is continued at room temperature for 16 hours. The resulting mixture is acidified with dilute hydrochloric acid, then ethanol is added and the solution is evaporated *in vacuo*. The residue is dissolved in a small amount of hot water and triturated with ethanol until cloudiness persists. The mixture is allowed to cool with separation of crystals. The crystals are collected, washed twice with ethanol and ether and air dried to yield 1.82 g of 5-(*p*-nitro-2-sulfobenzamido)-*N*-(4,6,8-trisulfo-1-naphthyl) isophthalamic acid methyl ester, tetrasodium salt.

A solution of 1.6 g of the preceding product in 25.0 ml of 1*N* sodium hydroxide is stirred at room temperature for 3 hours. The solution is acidified with dilute hydrochloric acid, then ethanol is added and the solution is evaporated *in vacuo*. The residue is dissolved in hot water and triturated with ethanol until cloudy. The mixture is allowed to cool and the resulting precipitate is collected by filtration, washed twice with ethanol and ether and air dried to provide 1.34 g of 5-(4-nitro-2-sulfobenzamido)-*N*-(4,6,8-trisulfo-1-naphthyl)isophthalamic acid, tetrasodium salt.

A mixture of 1.15 g of the above compound, 160 ml of water and 115 mg of 10% palladium-on-carbon catalyst is hydrogenated in a Parr shaker for 3 hours. The resulting mixture is filtered through diatomaceous earth. Ethanol is added to the filtrate and the solution is evaporated. The residue is dissolved in a small amount of water, then ethanol is added with separation of a solid. The solid is collected and dried to yield 890 mg of the product of the Example as a white powder.

## EXAMPLE 30

## 5,5'-[Ureylenebis[(2-sulfo-4,1-phenylenecarbonyl)-imino]]bis[N-4,6,8-trisulfo-1-naphthylisophthalamic acid], octasodium salt

A solution of 690 mg of the product of Example 29 and 220 mg of anhydrous sodium carbonate in 20 ml of water is phosgenated at a slow rate until it is acidic to Congo Red indicator paper. The mixture is evaporated and the resi-

due is dissolved in a minimum amount of water, ethanol is added the the resulting solid is collected by filtration. The solid (620 mg) is dissolved in 6.0 ml of water and is phosgenated again as above in the presence of 220 mg of anhydrous sodium carbonate. Ethanol is added to the resulting mixture which is then evaporated. The residue is dissolved in water and reprecipitated with ethanol. This step is repeated, then the final product is collected, washed twice with ethanol and ether and dried to yield 290 mg of the product of the Example as a white powder.

EXAMPLE 31

5-Amino-3'-carboxy-5'-(4,6,8-trisulfo-1-naphthylcarbamoyl) isophthalanilic acid, trisodium salt

To a stirred solution of 1.8 g of 5-amino-N-4,6,8--trisulfo-1-naphthylisophthalamic acid methyl ester, trisodium salt (prepared as described in Example 29), 15.0 ml of water and 305 mg of anhydrous sodium carbonate is added 837 mg of 3-carbomethoxy-5-nitrobenzoyl chloride (prepared as in Example 29). Additional water is added to facilitate stirring. The mixture is stirred for 3 hours and is acidified with dilute hydrochloric acid to give complete solution. Ethanol is added to the solution with stirring to provide a white precipitate. The precipitate is collected, washed with ethanol and ether and air dried. The product is reprecipitated from water and ethanol, then is collected and is washed twice with ethanol and ether and dried to yield 1.68 g of 3'-carboxy-5-nitro-5'-(4,6,8-trisulfo-1-naphthylcarbamoyl)isophthalanilic acid dimethyl ester, trisodium salt.

A solution of the entire product above and 50.0 ml of 1N sodium hydroxide is stirred at room temperature for 3 hours. The solution is acidified with dilute hydrochloric acid, then absolute ethanol is added and the solution is evaporated. The residue is dissolved in hot water, triturated with ethanol and cooled. The precipitate is collected and washed twice with both ethanol and ether then is dried to afford 930 mg of 3'-carboxy-5'-(4,6,8-trisulfo-1-naphthylcarbamoyl)-5-nitroisophthalanilic acid, trisodium salt.

A mixture of 730 mg of the preceding compound, 100 ml of water and 75.0 mg of 10% palladium-on-carbon catalyst is hydrogenated on a Parr shaker for 4 hours. The resulting mixture is filtered through diatomaceous earth and the filtrate is evaporated. The residue is dissolved in hot water, then absolute ethanol is added and a precipitate is formed on cooling to room temperature. The precipitate is collected by filtration and is washed twice with both ethanol and ether. The material is dried to yield 520 mg of the product of the Example as a tan powder.

EXAMPLE 32

5,5'-Ureylenebis[N-[3-carboxy-5-(4,6,8-trisulfo-1-naphthaylcarbamoyl)phenyl]isophthalamic acid], decasodium salt

A solution of 390 mg of the product of Example 31 and 55.0 mg of sodium carbonate in 10 ml of water is phosgenated until it becomes acidic. The resulting milky solution is neutralized with sodium carbonate and is evaporated. The residue is dissolved in water and absolute ethanol is added to provide a precipitate. The precipitate is collected and washed twice with both ethanol and ether, then is dried to yield 261 mg of the product of the Example as a white powder.

EXAMPLE 33

5,5'-Ureyelenbis[N-[3-carboxy-5-(4,6,8-trisulfo-1-naphthylcarbamoyl)phenyl]isophthalamic acid]hexasodium salt

A 125 mg portion of the product of Example 32 is dissolved in 5 ml of water and is then acidified with glacial acetic acid. Absolute ethanol is added to precipitate a product. The product is collected by filtration, then is washed with ethanol and ether and is dried to yield 92 mg of the product of the Example as a pale yellow powder.

EXAMPLE 34

5-Amino-3'-carboxy-5'-(3,5,8-trisulfo-1-naphthylcarbamoyl) isophthalanilic acid, trisodium salt

Following the procedure of Example 31, employing 5-amino-N-3,6,8-trisulfo-1-naphthylisophthalamic acid methyl ester, trisodium salt (prepared as described in Examples 3 and 5 using 8-amino-1,3,6-naphthalenetrisulfonic acid, trisodium salt) provides the product of the Example.

EXAMPLE 35

5,5'-Ureylenebis[N-[3-carboxy-5-(3,6,8-trisulfo-1-naphthylcarbamoyl)phenyl]isophthalamic acid], decasodium salt

Following the procedure of Example 32, phosgenation of the product of Example 34, provides the product of the Example.

EXAMPLE 36

5-Amino-3'-sulfo-5'-(4,6,8-trisulfo-1-naphthylcarbamoyl)isophthalanilic acid, tetrasodium salt

Following the procedure of Example 26, 4-nitro-2-sulfobenzoic acid anhydride is reacted with 8-amino-1,3,5--naphthalenetrisulfonic acid, trisodium salt, the product therefrom being reduced to provide 8-(4-amino-2-sulfobenzamido)-1,3,5-naphthalenetrisulfonic acid, tetrasodium salt. Reaction of the latter with 3-carbomethoxy-5-nitrobenzoyl chloride (prepared as described in Example 29), followed by hydrolysis and reduction gives the product of the Example.

EXAMPLE 37

5,5"-Ureylenebis[3'-sulfo-4'-(4,6,8-trisulfo-1-naphthylcarbamoyl)isophthalanilic acid], decasodium salt

Following the procedure of Example 32, phosgenation of the product of Example 36, provides the product of the Example.

EXAMPLE 38

8-[5-(4-Amino-2-sulfobenzamido)-o-toluenesulfonamido]-1,3,6--naphthalenetrisulbonic acid, tetrasodium salt

To a boiling solution of 100 g of 5-nitro-o-toluenesulfonic acid in 110 ml of water is added a solution of 53.6 g of sodium chloride in 150 ml of boiling water. The reaction mixture solidifies and is heated to boiling with the addition of sufficient water to provide solution. Then some of the water is boiled off and the mixture is allowed to stand for 16 hours. The solid formed is collected and dried to yield 92.5 g of 5-nitro-o-toluenesulfonic acid sodium salt.

A mixture of 50.0 g of the above compound, 125 ml of thionyl chloride and 1.3 ml of dimethylformamide is

stirred and refluxed for 3 hours. The excess thionyl chloride is distilled off and the residue is reevaporated twice with ether. The residue is extracted with ether and methylene chloride. The extracts are evaporated and the residue is dissolved in ether and is filtered. The filtrate is concentrated while adding petroleum ether, then is placed in an icebox for 16 hours. The solid formed is collected and dried to yield 33.4 g of 5-nitro-o-toluenesulfonyl chloride.

A mixture of 17.0 g of 8-amino-1,3,6-naphthalenetrisulfonic acid, trisodium salt, 17.5 g of the preceding compound and 7.8 g of anhydrous sodium carbonate in 210 ml of water is stirred at room temperature for 18 hours, then an additional 0.5 g of sodium carbonate and 1.0 g of 5-nitro-o-toluenesulfonyl chloride is added and stirring is continued for 18 hours longer. The reaction mixture is evaporated and 100 ml of water is added with stirring. The mixture is filtered and 900 ml of absolute ethanol is added to the filtrate with stirring. The mixture is stirred for 2 hours, then the precipitate is collected and is washed with ethanol and ether and is dried to yield 20.8 g of 8-(5-nitro-o-toluenesulfonamido)-1,3,6-naphthalenetrisulfonic acid, trisodium salt.

A mixture of 20.0 g of the above product, 90.0 ml of water and 2.0 g of 10% palladium-on-carbon catalyst is hydrogenated as described in Example 1. The reaction mixture is filtered through diatomaceous earth and the filtrate is evaporated. The residue is dissolved in a minimum amount of water, then is added dropwise to 800 ml of stirred absolute ethanol. The mixture is stirred for 2 hours and is allowed to stand for 48 hours. A light yellow solid is collected, washed with ethanol and ether, then is dried to yield 15.0 g of 8-(5-amino-o-toluenesulfonamido)-1,3,6-naphthalenetrisulfonic acid, trisodium salt.

A solution of 100 g of 5-nitro-o-toluenesulfonic acid in 600 ml of water plus 80 ml of 5N sodium hydroxide is heated to 90°C in a 2 liter Erlenmeyer flask, then 240 g

of potassium permanganate is added portionwise to maintain reflux over one hour and 15 minutes. The mixture is filtered and the residue is washed with water. The combined filtrate and washings are concentrated *in vacuo* and allowed to crystallize to give 86.2 g of crude 4-nitro-2-sulfobenzoic acid, sodium potassium salt. Recrystallization from water gives 71.3 g of purified product.

The total product above is dissolved in 250 ml of water plus 35 ml of concentrated hydrochloric acid by warming on a steam bath. The solution is then diluted with 300 ml of ethyl alcohol and allowed to crystallize at room temperature. The mixture is allowed to stand 48 hours in a chilled room, then is filtered. The precipitate is washed with cold 50% aqueous ethanol, then with ethanol and ether. The material is recrystallized from 200 ml of water and is dried at 110°C to give 52.0 g of 4-nitro-2-sulfobenzoic acid--2-sodium salt.

A 50.0 g portion of the preceding compound and 500 g of thionyl chloride is stirred and refluxed for 19 hours. The mixture is evaporated *in vacuo* and the residue is warmed with 300 ml of toluene and is filtered. The filtrate is concentrated *in vacuo* and the product is crystallized twice from toluene to give 30.4 g of 4-nitro-2-sulfobenzoic acid anhydride.

To a stirred solution of 9.0 g of 8-(5-amino-*o*--toluenesulfonamido)-1,3,6-naphthalenetrisulfonic acid, trisodium salt, and 3.34 g of sodium acetate trihydrate in 47.0 ml of water chilled to 0°C in an ice bath is added 4.29 g of 4-nitro-2-sulfobenzoic acid anhydride in one portion. The mixture is stirred for a total of 10 minutes in the ice bath and the undissolved material is separated. The solution, at 0°C, is acidified with 1.38 ml of concentrated hydrochloric acid, then is added dropwise, with vigorous stirring, to 750 ml of absolute ethanol. The product is collected, washed with 85% aqueous ethanol, ehtanol and ether and is dried to yield 11.75 g of 8-[5-(4-nitro-2--sulfobenzamido)-*o*-toluenesulfonamido]-1,3,6-naphthalenetrisulfonic acid, tetrasodium salt.

A mixture of 9.7 g of the preceding product, 80.0 ml of water and 1.0 g of 10% palladium-on-carbon catalyst is hydrogenated as previously described. The mixture is filtered and the filtrate is evaporated, dissolved in water and added dropwise to 650 ml of ethanol. The resulting mixture is stirred for 2 hours, then the product is separated and washed with ethanol and ether and dried to give 8.1 g of the product of the Example.

EXAMPLE 39

8-[5-(4-Amino-2-sulfobenzamido)-o-toluenesulfonamido]-1,3,5-naphthalenetrisulfonic acid, tetrasodium salt

Following the procedure of Example 38, employing 8-amino-1,3,5-naphthalenetrisulfonic acid, trisodium salt provides the product of the Example.

EXAMPLE 40

8,8'-[Ureylenebis[(2-sulfo-4,1-phenylenecarbonyl)-imino(6-methyl-3,1-phenylenesulfonyl)imino]]di-1,3,6-naphthalenetrisulfonic acid, octasodium salt

A cooled solution of 4.0 g of the product of Example 38, and 2.49 ml of pyridine in 35.0 ml of water is phosgenated until acidic to Congo Red indicator paper. An additional 1.38 ml of pyridine is added and phosgene is bubbled in again until acidic to Congo Red indicator. The solution is neutralized with pyridine and poured into 650 ml of absolute ethanol with stirring. The mixture is stirred for 30 minutes, then the solid is separated, washed with ethanol and ether and dried. The material is dissolved in 30.0 ml of water. The solution is adjusted to pH 8.0 with 5N sodium hydroxide, then is neutralized with glacial acetic acid and poured into 800 ml of absolute ethanol with stirring. The residue is dissolved in 25.0 ml of water and is added to 500 ml of absolute ethanol with stirring. An additional 300 ml of ethanol is added and the mixture is stirred for 16 hours, then is evaporated. The residue is dissolved in 35.0 ml of water, the solution is adjusted to pH 7.0 and is slowly added to 500 ml of absolute ethanol with stirring.

The solution is concentrated to 400 ml on a steam bath while adding water to provide a gum and a fluffy solid. The fluffy material is separated, washed with ethanol and ether and dried to yield 2.0 g of the product of the Example.

EXAMPLE 41

8,8'-[Ureylenebis[(2-sulfo-4,1-phenylenecarbonyl)imino(6-methyl-3,1-phenylenesulfonyl)imino]]di-1,3,5-naphthalenetrisulfonic acid, octasodium salt

Following the procedure of Example 40, phosgenation of the product of Example 39 provides the product of the Example.

EXAMPLE 42

8-[5-(4-Amino-2-sulfobenzamido)-2,4-xylenesulfonamido]-1,3,6-naphthalenetrisulfonic acid, tetrasodium salt

A 42.3 g amount of 2,4-dimethyl-5-nitrobenzenesulfonic acid in 50 ml of water is heated to 70°C, then a solution of 25.0 g of sodium chloride in 70.0 ml of water is added forming a solid. Water is added to a total volume of 300 ml and the mixture is heated to boiling. The solution is filtered and the filter is washed with 100 ml of hot water which is added to the filtrate. The filtrate is concentrated to 300 ml and is allowed to cool. The solution is placed in an icebox for 16 hours with formation of a crystalline precipitate. The crystals are collected by filtration to yield 34.6 g of product. The filtrate is concentrated to 150 ml and is cooled and filtered to provide an additional 4.2 g of product. The fractions are combined to provide 38.8 g of 5-nitro-2,4-xylenesulfonic acid, sodium salt.

A mixture of the entire product above, 100 ml of thionyl chloride and 1.0 ml of dimethylformamide (dried over molecular sieves) is heated at reflux for 3 hours. The mixture is allowed to cool at room temperature, then is evaporated *in vacuo*. Ether is added and the mixture is re-evaporated. The residue is extracted several times with boiling ether. The ether extracts are concentrated to 300 ml and allowed to cool. The crystals formed are collected by filtration to provide 2.0 g of product. The filtrate is evaporated and the residue is extracted once with boiling methylene chloride. The extract is evaporated and the residue is dissolved in ether, concentrated to a small volume and cooled. The crystalline precipitate is collected to yield 22.0 g of additional product. The fractions are combined to provide 24.0 g of 5-nitro-2,4-xylenesulfonyl chloride.

A mixture of 11.0 g of 8-amino-1,3,6-naphthalenetrisulfonic acid, trisodium salt, 14.0 g of the preceding product, 6.15 g of anhydrous sodium carbonate and 140 ml of water is stirred with heat until thin layer chromatography shows the reaction is complete. The mixture is filtered and the filtrate is concentrated in vacuo removing the discarding crops of solid as they precipitate. The final filtrate is evaporated, the residue is dissolved in water, absolute ethanol is added and the mixture is stirred for one hour and filtered. The solid is washed with ethanol and ether giving 9.8 g of 8-(5-nitro-2,4-xylenesulfonamido)-1,3,6-naphthalenetrisulfonic acid, trisodium salt. An additional 1.28 g of the same product is derived from second and third crops of the filtrate.

A mixture of 11.0 g of the above product, 50.0 ml of water and 1.0 g of 10% palladium-on-carbon catalyst is hydrogenated as described in Example 1. The reaction mixture is filtered through diatomaceous earth and the filter is washed with water. The filtrate is evaporated, the residue is dissolved in 30 ml of water and is added to 450 ml of absolute ethanol. The mixture is stirred for 1/2 hour and the product is separated by filtration. An additional crop of product is derived from re-precipitation of the filtrate. The combined product is washed with ether and dried to yield 7.52 g of 8-(5-amino-2,4-xylenesulfonamido)-1,3,6-naphthalenetrisulfonic acid, trisodium salt.

To a stirred solution of 5.0 g of the product above and 1.81 g of sodium acetate trihydrate in 26.0 ml of water chilled to 0°C in an ice bath is added 2.29 g of 4-nitro-2-sulfobenzoic acid anhydride in one portion. The mixture is stirred for a total of 10 minutes in the ice bath

and the undissolved material is separated. The solution at 0°C is acidified with 0.74 ml of concentrated hydrochloric acid and 200 ml of absolute ethanol is added to precipitate a solid. The product is separated by filtration, washed with ethanol and ether and dried. The filtrate is evaporated and the resulting residue is dissolved in a minimum amount of water, then absolute ethanol is added to precipitate additional product. The product fractions are combined to provide 5.84 g of 8-[5-(4-nitro-2-sulfobenzamido)-2,4-xylenesulfonamido]-1,3,6-naphthalenetrisulfonic acid, tetrasodium salt.

A mixture of 5.0 g of the preceding product, 50.0 ml of water and 700 mg of 10% palladium-on-carbon catalyst is hydrogenated as previously described. The reaction mixture is filtered through diatomaceous earth and the filtrate is evaporated. The residue is dissolved in a minimum amount of water and is added slowly to 250 ml of absolute ethanol with stirring. The precipitate is collected, washed with ethanol and ether and dried to yield 4.05 g of the product of the Example.

EXAMPLE 43

8-[5-(4-Amino-2-sulfobenzamido)-2,4-xylenesulfonamido-1,3,5-
-naphthalenetrisulfonic acid, tetrasodium salt

Following the procedure of Example 42, employing 8-amino-1,3,5-naphthalenetrisulfonic acid, trisodium salt provides the product of the Example.

EXAMPLE 44

8,8'-[Ureylenebis[(2-sulfo-4,1-phenylenecarbonyl)imino(4,6-
-dimethyl-3,1-phenylenesulfonyl)imino]]di-1,3,6-naphthalenetrisulfonic acid, octasodium salt

A cooled solution of 4.0 g of the product of Example 42 and 2.35 ml of pyridine in 35.0 ml of water is phosgenated until acidic to Congo Red indicator paper. An additional 1.1 ml of pyridine is added and phosgene is bubbled in again until acidic to Congo Red indicator. The solution is neutralized with pyridine and poured into 650 ml of absolute ethanol with stirring. The mixture is stirred for

30 minutes, then the solid is separated, washed with ethanol and ether and dried. The crude product is dissolved in 30.0 ml of water and is adjusted to pH 8.0 with 5N sodium hydroxide. The solution is neutralized to pH 7.0 with glacial acetic acid and is poured into 650 ml of absolute ethanol. The mixture is stirred for one hour, then is evaporated. The residue is dissolved in 30.0 ml of water, several drops of glacial acetic acid are added and the solution is added to 800 ml of absolute ethanol with stirring. The mixture is concentrated to 400 ml on a steam bath while adding water, with separation of an oil. The aqueous solution is decanted from the oil and ethanol is added to it to provide a fine precipitate. The precipitate is separated, washed with ethanol and ether and dried to yield 2.15 g of the product of the Example.

EXAMPLE 45

8,8'-[Ureylenebis[(2-sulfo-4,1-phenylenecarbonyl)imino(4,6-dimethyl-3,1-phenylenesulfonyl)imino]]-di-1,3,5-naphthalenetrisulfonic acid, octasodium salt

Following the procedure of Example 44, phosgenation of the product of Example 43 provides the product of the Example.

EXAMPLE 46

5-Amino-2'-methyl-5'-(4,6,8-trisulfo-1-naphthylsulfamoyl)-isophthalanilic acid, trisodium salt

A mixture of 25.0 g of p-toluenesulfonic acid and 95.0 ml of concentrated nitric acid is heated on a steam bath for 30 minutes. The solution is poured into 250 ml of water and is evaporated at reduced pressure. A small quantity of water is added and the mixture is evaporated again. This step is repeated two additional times to remove all of the nitric acid. The mixture is neutralized with a saturated solution of sodium carbonate and is evaporated affording a yellow solid. The solid is slurried with absolute ethanol, is collected by filtration and is washed twice with both ethanol and ether to give 10.3 g of product. The filtrate is allowed to stand and the solid formed is

collected and washed as above to provide 10.0 g of additional product and a total of 20.3 g of 3-nitro-p-toluenesulfonic acid, sodium salt.

A mixture of 20.0 g of the above compound, 250 ml of thionyl chloride and 20.0 ml of dimethylformamide is refluxed for 16 hours. The excess thionyl chloride is removed by distillation, then the mixture is cooled and ether is added. The mixture is evaporated and the crude product is distilled under vacuum. The fraction recovered at a pressure of 1.0-1.5 mm of mercury and a boiling point of 152°-154°C provides 11.5 g of 3-nitro-p-toluenesulfonyl chloride.

An 8.0 g portion of the preceding product is added to a stirred solution of 7.2 g of 8-amino-1,3,5-naphthalenetrisulfonic acid, trisodium salt and 1.7 g of anhydrous sodium carbonate in 30.0 ml of water with separation of an oil. The mixture is stirred for 16 hours and then is evaporated. The residue is dissolved in water and absolute ethanol is added to provide a precipitate. The product is collected and washed with ethanol and ether. The filtrate is evaporated and the residue is precipitated as above to provide additional product. A third crop is obtained from the final filtrate to provide a total of 8.6 g of 8-(3-nitro-p-toluenesulfonamido)-1,3,5-naphthalenetrisulfonic acid, trisodium salt.

A mixture of 8.0 g of the above product, 160 ml of water and 800 mg of 10% palladium-on-carbon catalyst is hydrogenated on a Parr shaker until no additional hydrogen is absorbed. The resulting mixture is filtered through diatomaceous earth and the filtrate is evaporated. The residue is dissolved in hot water and is filtered. The filtrate is triturated with ethanol until cloudiness persists, then is allowed to stand at room temperature for 16 hours. The mixture is filtered and the filtrate is evaporated to afford a gummy material which is dissolved in a small amount of water and triturated with ethanol. An additional 200 ml of ethanol is added and the mixture is stirred for one hour to provide a solid. The solid is collected and washed with ethanol

and ether to yield 4.6 g of 8-(3-amino-p-toluenesulfonamido)--1,3,5-naphthalenetrisulfonic acid, trisodium salt.

To a stirred solution of 2.5 g of the above product, 20 ml of water and 428 mg of anhydrous sodium carbonate is added 1.2 g of 3-carbomethoxy-5-nitrobenzoyl chloride (prepared as described below). The mixture is stirred at room temperature for 16 hours, then is evaporated. The residue is dissolved in hot water and is filtered. The filtrate is triturated with absolute ethanol and is allowed to cool forming a gelatinous mass. The material is collected and washed with ethanol and ether to yield 2.5 g of 2'-methyl-5'-nitro-(4,6,8-trisulfo--1-naphthylsulfamoyl)isophthalanilic acid, methyl ester, trisodium salt as a light tan powder.

To a stirred solution of 2.25 g of the above product in 20 ml of water is added 3.0 ml of 1N sodium hydroxide. The mixture is stirred at room temperature for 16 hours, then is acidified with glacial acetic acid and is evaporated. The resulting solid is dissolved in hot water, then is triturated with ethanol until crystllization occurs. The mixture is cooled, the precipitate is collected and washed with ethanol and ether. The product is reprecipitated from water with ethanol, then is collected and is washed as above and dried to yield 1.52 g of 2'-methyl-5-nitro-5'-[(4,6,8-trisulfo-1--naphthyl)sulfamoyl]isophthalanilic acid, trisodium salt.

A mixture of 1.25 g of the preceding product, 100 ml of water and 140 mg of 10% palladium-on-carbon catalyst is hydrogenated in a Parr shaker until no additional hydrogen is absorbed. The resulting mixture is filtered, and the filtrate is evaporated. The residue is dissolved in hot water and ethanol is added. The mixture is filtered and the filtrate is evaporated yielding a yellow-white powder. The powder is washed with ether and dried to provide 950 mg of the product of the Example.

<u>Preparation of 3-carbomethoxy-5-nitrobenzoyl chloride</u>

A mixture of 7.46 g of potassium hydroxide in 87.5 ml of methanol is added to a stirred solution of 3.175 g of the preceding product in 331.0 ml of acetone. A solid is

precipitated and stirring is continued for 16 hours. The solid (A) is filtered off, washed with ether and set aside. The filtrate is evaporated, the residue is extracted with 125 ml of warm water and is filtered. The filtrate is acidified with dilute hydrochloric acid to produce a precipitate which is collected and dried to yield 3.4 g of product. The solid (A) is extracted with 250 ml of warm water and is filtered. The filtrate is filtered again at room temperature, acidified with dilute hydrochloric acid and cooled. The precipitate is collected and dried to give 18.25 g of additional product identified as 5-nitro-isophthalic acid, 3-methyl ester.

A mixture of 18.38 g of the above product, 60 ml of thionyl chloride and 0.37 ml of dimethylformamide is heated at 60°C for 2.5 hours. The solution is evaporated, then is treated with toluene, and again is evaporated. The residue is slurried in hot diethyl ether and the ether volume is reduced by evaporation. The mixture is chilled and filtered. The precipitate is washed with cold ether and is dried. The material is extracted with 500 ml of boiling hexane by decantation. The hexane is cooled and filtered to yield 14.1 g of 3-carbomethoxy-5-nitrobenzoyl chloride.

EXAMPLE 47

5-Amino-2'-methyl-5'-(3,6,8-trisulfo-1-naphthylsulfamoyl)-isophthalanilic acid, trisodium salt

Following the procedure of Example 46 employing 8-amino-1,3,6-naphthalenetrisulfonic acid, trisodium salt yields the product of the Example.

EXAMPLE 48

5,5"-Ureylenebis[2'-methyl-5'-(4,6,8-trisulfo-1-naphthylsulfamoyl)isophthalanilic acid], octasodium salt

A solution of 750 mg of the product of Example 46 10 ml of water and 102 mg of anhydrous sodium carbonate is phosgenated until acidic to Congo Red indicator paper. The solution is neutralized with sodium carbonate, then phosgenated again until acidic. The solution is neutralized as above and then acidified with glacial acetic acid. The reaction mixture is

evaporated and the residue is dissolved in water. Ethanol is added providing an oil. The aqueous solution is decanted and ethanol is added again to separate additional oil. The solution is decanted again, ethanol is added and the resulting precipitate is filtered, washed with ethanol and ether and dried affording 400 mg of the product of the Example.

EXAMPLE 49

5,5"-Ureylenebis[2'-methyl-5'-(3,6,8-trisulfo-1-naphthylsulfamoyl)isophthalanilic acid], octasodium salt

Following the procedure of Example 48, phosgenation of the product of Example 47 provides the product of the Example.

EXAMPLE 50

8-[$N^3$-(4-Amino-2-sulfobenzoyl)metanilamido]-1,3,6-naphthalenetrisulfonic acid, tetrasodium salt

To a stirred solution of 21.9 g of 8-amino-1,3,6-naphthalenetrisulfonic acid, trisodium salt and 11.4 g of anhydrous sodium carbonate in 280 ml of water is added 24.0 g of m-nitrobenzenesulfonyl chloride. The mixture is stirred at room temperature for 16 hours, then an additional 1.0 g of sodium carbonate and 2.0 g of m-nitrobenzenesulfonyl chloride are added and stirring is continued for 3 hours longer. The mixture is evaporated and the residue is dissolved in 200 ml of water. A copious amount of absolute ethanol is added and the solid formed is collected and washed with ethanol and ether, then is dried to yield 26.1 g of 8-m-nitrobenzenesulfonamido-1,3,6-naphthalenetrisulfonic acid, trisodium salt.

A mixture of 26.1 g of the preceding product, 175 ml of water and 2.09 g of palladium-on-carbon catalyst is hydrogenated in a Parr shaker until no additional hydrogen is absorbed. The resulting mixture is filtered through diatomaceous earth and the filtrate is evaporated. The residue is dissolved in 60.0 ml of water and, with stirring, 400 ml of absolute ethanol is added to precipitate a solid. The mixture is allowed to stir for 2 hours, then is filtered.

The product is washed with absolute ethanol and ether to give 25.3 g of 8-metanilamido-1,3,6-naphthalenetrisulfonic acid, trisodium salt.

To a stirred and chilled solution (ice-bath at 0°C) of 9.0 g of the above product and 3.42 g of sodium acetate trihydrate in 48.0 ml of water is added, in one portion, 4.35 g of 4-nitro-2-sulfobenzoic acid anhydride. The mixture is stirred for a total of 10 minutes at 0°C. The undissolved material is removed by filtration and the filtrate in the ice-bath is acidifi-d with 1.41 ml of concentrated hydrochloric acid, then 400 ml of absolute ethanol is added to separate the product. The material is collected by filtration and is washed with 85% aqueous ethanol, then ethanol and ether to yield 3.87 g of product. Additional product (5.3 g) is recovered by adding ethanol to the filtrate above, the filtering and washing as described. The total yield is 9.1 g of 8-[$\underline{N}^3$-(4-nitro-2-sulfobenzoyl)metanilzmido]-1,3,6--naphthalenetrisulfonic acid, tetrasodium salt.

A mixture of 11.1 g of the preceding compound (prepared as described above), 90.0 ml of water and 1.0 g of 10% palladium-on-carbon catalyst is hydrogenated in a Parr shaker until no additional hydrogen is absorbed. The resulting mixture is filtered and the filtrate is evaporated to a glass. The glass is triturated in hot ethanol, then is collected by filtration and is washed with ethanol and ether and dried to yield 10.58 g of the product of the Example.

EXAMPLE 51

8,8'-[Ureylenebis[(2-sulfo-1,4-phenylenecarbonyl)-imino(3,1--phenylenesulfonyl)imino]]di-1,3,6-naphthalenetrisulfonic acid, octasodium salt

A solution of 5.0 g of the product of Example 50 and 3.0 ml of pyridine in 40 ml of water is phosgenated until it is acidic to Congo Red indicator paper. An additional 2.0 ml of pyridine is added and phosgene is bubbled in again until the solution is acidic. The solution is neutralized with pyridine and is added to 400 ml of absolute ethanol with stirring to provide a gum. The supernatant is decanted, and

fresh ethanol is added to the gum. The mixture is stirred vigorously for 2 hours, then is filtered. The material on the filter is washed with ethanol and ether and is air dried, then is dissolved in 25.0 ml of water and basified to pH (8-9) with 5N sodium hydroxide. The solution is neutralized with glacial acetic acid and filtered. The filtrate is added to 400 ml of absolute ethanol with stirring to precipitate a light pink solid. The solid is separated and is washed with ethanol and ether and is dried to yield 4.0 g of the product of the Example.

EXAMPLE 52

8-(3-Metanilamido-p-toluenesulfonamido)-1,3,5-naphthalenetrisulfonic acid, trisodium salt

A mixture of 25.0 g of p-toluenesulfonic acid and 95.0 ml of concentrated nitric acid is heated on a steam bath for 30 minutes. The solution is poured into 250 ml of water and evaporated at reduced pressure. A small quantity of water is added and the mixture is evaporated again. This step is repeated two additional times to remove all of the nitric acid. The mixture is neutralized with a saturated solution of sodium carbonate and evaporated affording a yellow solid. The solid is slurried with absolute ethanol, collected by filtration and washed twice with both ethanol and ether to give 10.3 g of product. The filtrate is allowed to stand and the solid formed is collected and washed as above to provide 10.0 g of additional product and a total of 20.3 g of 3-nitro-p-toluenesulfonic acid, sodium salt.

A mixture of 20.0 g of the above compound, 250 ml of thionyl chloride and 20.0 ml of dimethylformamide is refluxed for 16 hours. The excess thionyl chloride is removed by distillation, then the mixture is cooled and ether is added. The mixture is evaporated and the crude product is distilled under vacuum. The fraction recovered at a pressure of 1.0-1.5 mm of mercury and a boiling point of 152°-154°C provides 11.5 g of 3-nitro-p-toluenesulfonyl chloride.

To a warm solution of 106.4 g of (80.5%) 8-amino-1,3,5-naphthalenetrisulfonic acid in 100 ml of water and

45.0 ml of 5N sodium hydroxide is slowly added 500 ml of absolute ethanol with vigorous stirring for 30 minutes. The mixture is cooled to room temperature and filtered. The precipitate is washed with 80% aqueous ethanol, ethanol and ether and dried *in vacuo* at 110°C for 16 hours to give 103.7 g of 8-amino-1,3,5-naphthalenetrisulfonic acid, trisodium salt.

An 8.0 g portion of 3-nitro-*p*-toluenesulfonyl chloride is added to a stirred solution of 7.2 g of 8-amino-1,3,5-naphthalenetrisulfonic acid trisodium salt, and 1.7 g of anhydrous sodium carbonate in 30.0 ml of water with separation of an oil. The mixture is stirred for 16 hours and then is evaporated. The residue is dissolved in water and absolute ethanol is added to provide a precipitate. The product is collected and washed with ethanol and ether. The filtrate is evaporated and the residue is precipitated as above to provide additional product. A third crop is obtained from the final filtrate to provide a total of 8.6 g of 8-(3-nitro-*p*-toluenesulfonamido)-1,3,5-naphthalenetrisulfonic acid, trisodium salt.

A mixture of 8.0 g of 8-(3-nitro-*p*-toluenesulfonamido)-1,3,5-naphthalene trisulfonic acid trisodium salt, 160 ml of water and 800 mg of 10% palladium on carbon catalyst is hydrogenated on a Parr shaker until no additional hydrogen is absorbed. The resulting mixture is filtered through diatomaceous earth and the filtrate is evaporated. The residue is dissolved in hot water and filtered. The filtrate is triturated with ethanol until cloudiness persists, then is allowed to stand at room temperature for 16 hours. The mixture is filtered and the filtrate evaporated to afford a gummy material which is dissolved in a small amount of water and triturated with ethanol. An additional 200 ml of ethanol is added and the mixture is stirred for one hour to provide a solid. The solid is collected and washed with ethanol and ether to yield 4.6 g of 8-(3-amino-*p*-toluenesulfonamido)-1,3,5-naphthalenetrisulfonic acid, trisodium salt as a light tan powder.

To a stirred solution of 2.0 g of 8-(3-amino-*p*-

-toluenesulfonamido)-1,3,5-naphthalenetrisulfonic acid trisodium salt and 342 mg of anhydrous sodium carbonate in 10 ml of water is added 1.5 g of m-nitrobenzenesulfonyl chloride. The mixture is stirred for 16 hours. An additional 170 mg of anhydrous sodium carbonate is added followed by 750 mg of m-nitrobenzenesulfonyl chloride and the mixture is stirred for an additional 16 hours. The reaction mixture is evaporated and the residue dissolved in hot water. A product is precipitated on the addition of absolute ethanol. The product is collected, washed with ethanol and ether and dried. Additional product is collected from the filtrate and is washed and dried as above. The above fractions of product are combined and dissolved in water, then 2.5 ml of 5N sodium hydroxide is added and the mixture is stirred for 30 minutes. The mixture is acidified with glacial acetic acid and evaporated. The residue is dissolved in hot water and precipitated with ethanol. The product is collected and washed twice with ethanol and ether and dried to yield 700 mg of 8-(3-m-nitrobenzenesulfonamido-p-toluenesulfonamido)-1,3,5-naphthalenetrisulfonic acid, trisodium salt.

A mixture of 550 mg of 8-(3-m-nitrobenzenesulfonamido-p-toluenesulfonamido)-1,3,5-naphthalenetrisulfonic acid, trisodium salt, 30.0 ml of water and 65.0 mg of 10% palladium on carbon catalyst is hydrogenated on a Parr shaker for 1.75 hours. The resulting mixture is filtered through diatomaceous earth and the filtrate is evaporated. The crude material is dissolved in a minimum of hot water and triturated with ethanol. The resulting solid (A) is collected by filtration and washed with ethanol and ether. The filtrate is evaporated and the residue dissolved in water. Ethanol is added and the solution is evaporated to yield a white powder (B). Fractions (A) and (B) are combined and dried to yield 500 mg of the desired product.

## EXAMPLE 53

### 8,8'-[Ureylenebis[(m-phenylenesulfonylimino)(4-methyl-3,1-phenylenesulfonyl)imino]]di-1,3,5-naphthalenetrisulfonic acid, hexasodium salt

Phosgene gas is bubbled into a solution of 400 mg of the product of Example 52, 20.0 ml of water and 55.0 mg of anhydrous sodium carbonate until the reaction mixture is acidic to Congo Red indicator. The solution is neutralized with sodium carbonate then an additional 55.0 mg of sodium carbonate is added and phosgenation is repeated until the reaction mixture is acidic. The solution is neutralized with sodium carbonate and the excess sodium carbonate is decomposed with acetic acid. The reaction mixture is evaporated and the residue is dissolved in hot water and filtered. The filtrate is triturated with ethanol and allowed to cool. The precipitate formed is collected and washed with ethanol and ether then is re-precipitated from water and ethanol and collected and washed as above. The product is dried to yield 336 mg of the desired product as a tan powder.

EXAMPLE 54

8-(3-Metanilamido-p-toluenesulfonamido)-1,3,6-naphthalenetrisulfonic acid, trisodium salt

Following the procedure of Example 52, employing 8-amino-1,3,6-naphthalenetrisulfonic acid provides the product of the Example.

EXAMPLE 55

8,8'-[Ureylenebis[(m-phenylenesulfonylimino)(4-methyl-3,1-phenylsulfonyl)imino]]di-1,3,6-naphthalenetrisulfonic acid, hexasodium salt

Following the procedure of Example 53, phosgenation of the product of Example 54, provides the product of the Example.

EXAMPLE 56

8-[$N^3$-(m-Aminophenylsulfonyl)metanilamido]-1,3,6-naphthalenetrisulfonic acid, trisodium salt

To a stirred solution of 21.9 g of 8-amino-1,3,6-naphthalenetrisulfonic acid, trisodium salt and 11.4 g of anhydrous sodium carbonate in 280 ml of water is added 24.0 g of m-nitrobenzenesulfonyl chloride. The mixture is stirred at room temperature for 16 hours, then an additional 1.0 g of sodium carbonate and 2.0 g of m-nitrobenzenesulfonyl chloride are added and stirring is continued for 3 hours longer. The mixture is evaporated and the residue is dissolved in 200 ml of water. A copious amount of absolute ethanol is added and the solid formed is collected and washed with ethanol and ether, then is dried to yield 26.1 g of 8-m-nitrobenzenesulfonamido-1,3,6-naphthalenetrisulfonic acid, trisodium salt.

A mixture of 26.1 g of 8-m-nitrobenzenesulfonamido-1,3,6-naphthalenetrisulfonic acid, trisodium salt, 175 ml of water and 2.09 g of palladium on carbon catalyst is hydrogenated in a Parr shaker until no additional hydrogen is absorbed. The resulting mixture is filtered through diatomaceous earth and the filtrate is evaporated. The residue is dissolved in 60.0 ml of water and, with stirring, 400 ml of absolute ethanol is added to precipitate a solid. The mixture is allowed to stir for 2 hours, then is filtered. The product is washed with absolute ethanol and ether to give 25.3 g of 8-metanilamido-1,3,6-naphthalenetrisulfonic acid, trisodium salt.

To a stirred solution of 11.26 g of 8-metanilamido-1,3,6-naphthalenetrisulfonic acid, trisodium salt and 4.72 g of anhydrous sodium carbonate in 200 ml of water is added 10.0 g of m-nitrobenzenesulfonyl chloride. The mixture is stirred for 18 hours and is filtered. A copious amount of absolute ethanol is added to the filtrate, with stirring, to provide a precipitate. The mixture is stirred for one hour, then the solid is separated and washed with absolute ethanol and ether to yield 8.9 g of 8-[$N^3$-(m-nitrophenylsulfonyl)metanilamido]-1,3,6-naphthalenetrisulfonic acid, trisodium salt.

A mixture of 8.9 g of 8-[$N^3$-(m-nitrophenylsulfonyl)-metanilamido]-1,3,6-naphthalenetrisulfonic acid, trisodium salt, 90.0 ml of water and 1.0 g of 10% palladium on carbon catalyst is hydrogenated as previously described. The resulting mixture is filtered through diatomaceous earth and the filtrate is evaporated. The residue is dissolved in 25.0 ml of water, then absolute ethanol is added to precipitate the

product. The precipitate is collected, is washed with ethanol and ether and dried to yield 6.1 g of the desired product.

EXAMPLE 57

8,8'-[Ureylenebis[[(1,3-phenylenesulfonylimino)-1,3--phenylenesulfonyl]imino]]di-1,3,6-naphthalenetrisulfonic acid, hexasodium salt

Phosgene is bubbled into a solution of 3.95 g of the product of Example 56 and 2.6 ml of pyridine in 35.0 ml of water until acidic to Congo Red indicator paper. An additional 1.5 ml of pyridine is added and phosgene is bubbled in again until acidic. The mixture is neutralized with pyridine and is poured into 450 ml of stirred absolute ethanol to provide a gum. The supernatant is decanted, the gum is triturated with additional absolute ethanol to yield a solid (A) (1.8 g) which is collected and washed with ethanol and ether. The supernatant above is evaporated. The residue is triturated with a copious amount of absolute ethanol and filtered. The solid (B) is washed with ethanol and ether to provide 2.5 g of material.

Fractions (A) and (B) above are combined and dissolved in 25.0 ml of water. The solution is basified to pH 8-9 with 5N sodium hydroxide then is neutralized with acetic acid. The solution is added dropwise to 400 ml of stirred absolute ethanol to yield a precipitate. Stirring is continued for one hour, then the precipitate is separated, washed with absolute ethanol and ether and dried to yield 2.0 g of the desired product.

EXAMPLE 58

8-[$N^3$-(m-Aminophenylsulfonyl)metanilamido]-1,3,5--naphthalenetrisulfonic acid, trisodium salt

Following the procedure of Example 56, employing 8-amino-1,3,5-naphthalenetrisulfonic acid, trisodium salt provides the product of the Example.

EXAMPLE 59

8,8'-[Ureylenebis[[(1,3-phenylenesulfonylimino)-1,3--phenylenesulfonyl]imino]]di-1,3,5-naphthalene-trisulfonic acid, hexasodium salt

Phosgenation (according to procedure of Example 57) of the product of Example 58 provides the product of the Example.

EXAMPLE 60

8-[5-(5-Amino-o-toluenesulfonamido)-o-toluenesulfonamido]-1,3,6-naphthalenetrisulfonic acid, trisodium salt

To a boiling solution of 100 g of 5-nitro-o-toluenesulfonic acid in 110 ml of water is added a solution of 53.6 g of sodium chloride in 150 ml of boiling water. The reaction mixture solidifies and is heated to boiling with the addition of sufficient water to provide solution. Then some of the water is boiled off and the mixture is allowed to stand for 16 hours. The solid formed is collected and dried to yield 92.5 g of 5-nitro-o-toluenesulfonic acid sodium salt.

A mixture of 50.0 g of 5-nitro-o-toluenesulfonic acid sodium salt, 125 ml of thionyl chloride and 1.3 ml of dimethylformamide is stirred and refluxed for 3 hours. The excess thionyl chloride is distilled off and the residue is reevaporated twice with ether. The residue is extracted with ether and methylene chloride. The extracts are evaporated and the residue is dissolved in ether and filtered. The filtrate is concentrated while adding petroleum ether, then is placed in an ice box for 16 hours. The solid formed is collected and dried to yield 33.4 g of 2-methyl-5-nitrobenzenesulfonyl chloride.

A mixture of 17.0 g of 8-amino-1,3,6-naphthalenetrisulfonic acid, trisodium salt, 17.5 g of 2-methyl-5-nitrobenzenesulfonyl chloride and 7.8 g of anhydrous sodium carbonate in 210 ml of water is stirred at room temperature for 18 hours, then an additional 0.5 g of sodium carbonate and 1.0 g of 2-methyl-5-nitrobenzenesulfonyl chloride is added and stirring is continued for 18 hours longer. The reaction mixture is evaporated and 100 ml of water is added with stirring. The mixture is filtered and 900 ml of absolute ethanol is added to the filtrate with stirring. The mixture is stirred for 2 hours, then the precipitate is collected, washed

with ethanol and ether and dried to yield 20.8 g of 8-(5-nitro-o-toluenesulfonamido)-1,3,6-naphthalenetrisulfonic acid, trisodium salt.

A mixture of 20.0 g of 8-(5-nitro-o-toluenesulfonamido)-1,3,6-naphthalenetrisulfonic acid, trisodium salt, 90.0 ml of water and 2.0 g of 10% palladium on carbon catalyst is hydrogenated as described in Example 1. The reaction mixture is filtered through diatomaceous earth and the filtrate is evaporated. The residue is dissolved in a minimum amount of water, then is added dropwise to 800 ml of stirred absolute ethanol. The mixture is stirred for 2 hours and allowed to stand for 48 hours. A light yellow solid is collected, washed with ethanol and ether, then is dried to yield 15.0 g of 8-(5-amino-o-toluenesulfonamido)-1,3,6-naphthalenetrisulfonic acid, trisodium salt.

A mixture of 7.0 g of 8-(5-amino-o-toluenesulfonamido)-1,3,6-naphthalenetrisulfonic acid, trisodium salt, 5.35 g of 2-methyl-5-nitrobenzenesulfonyl chloride and 2.5 g of anhydrous sodium carbonate in 120 ml of water is stirred at room temperature for 7 hours, then an additional 0.5 g of sodium carbonate and 1.0 g of 2-methyl-5-nitrobenzenesulfonyl chloride is added and stirring is continued. After 16 hours, 0.5 g of sodium carbonate and 1.0 g of acid chloride is added again and stirring is continued for several hours. The reaction mixture is filtered and the filtrate is concentrated and precipitated crops isolated as formed. The product crops are combined to yield 6.0 g of 8-[5-(5-nitro-o-toluenesulfonamido)-o-toluenesulfonamido]-1,3,6-naphthalenetrisulfonic acid, trisodium salt.

A mixture of 6.0 g of 8-[5-(5-nitro-o-toluenesulfonamido)-o-toluenesulfonamido]-1,3,6-naphthalenetrisulfonic acid, trisodium salt, 70.0 ml of water and 920 mg of 10% palladium on carbon catalyst is hydrogenated as previously described. The resulting mixture is filtered through diatomaceous earth and the filtrate is evaporated. The residue is dissolved in 30 ml of water and added with stirring to 300 ml of absolute ethanol forming a gum. The supernatant is decanted and the gum is triturated with ethanol to provide a solid which is collected by filtration and washed with ethanol and ether to yield 1.4 g of product. Additional product (1.5 g) is precipitated from the supernatant above and is collected and washed as above and the filtrates above are evaporated and triturated with ethanol to provide 1.8 g of product. The above fractions are combined and dried to yield 4.15 g of the desired product.

EXAMPLE 61

8,8'-[Ureylenebis[[[(6-methyl-3,1-phenylene)sulfonyl]-imino]-[[(6-methyl-3,1-phenylene)sulfonyl]imino]]]di-1,3,6-naphthalenetrisulfonic acid, hexasodium salt

Phosgene gas is bubbled through a solution of 4.0 g of the product of Example 60 and 2.54 ml of pyridine in 35.0 ml of water with vigorous stirring until the solution becomes acidic to Congo Red indicator paper. An additional 1.3 ml of pyridine is added and the solution is phosgenated again until acidic to Congo Red indicator. The mixture is neutralized with pyridine and poured into 650 ml of absolute ethanol with stirring. Stirring is continued for 30 minutes and the precipitate is collected and washed with ethanol and ether. The material is dried, then is dissolved in 20 ml of water. The solution is made alkaline (pH 8-9) with 5N sodium hydroxide, then neutralized with glacial acetic acid and added to 400 ml of absolute ethanol with stirring. The solution is concentrated to provide a precipitate. The solid is separated then washed with ethanol and ether and dried to yield 1.0 g of the desired product.

EXAMPLE 62

8-[5-(5-Amino-o-toluenesulfonamido)-o-toluenesulfonamido]-1,3,5-naphthalenetrisulfonic acid, trisodium salt

Following the procedure of Example 60, employing 8-amino-1,3,5-naphthalenetrisulfonic acid, trisodium salt provides the product of the Example.

EXAMPLE 63

8,8'-[Ureylenebis[[[(6-methyl-3,1-phenylene)sulfonyl]-imino]-[[(6-methyl-3,1-phenylene)sulfonyl]imino]]]di--1,3,5-naphthalenetrisulfonic acid, hexasodium salt

Following the phosgenation procedure of Example 61, the amine of Example 62 is converted into the product of the Example.

EXAMPLE 64

8-[3-(3-Amino-p-toluamido)-p-toluenesulfonamido]--1,3,5-naphthalenetrisulfonic acid, trisodium salt

Following the procedure of Example 52, reaction of 8-(3-amino-p-toluenesulfonamido)-1,3,5-naphthalenetrisulfonic acid, trisodium salt with 3-nitro-p-toluoyl chloride followed by reduction with palladium on carbon catalyst provides the product of the Example.

EXAMPLE 65

8,8'-[Ureylenebis[[[(4-methyl-3,1-phenylenecarbonyl)-imino](4-methyl-3,1-phenylenesulfonyl)]imino]]di--1,3,5-naphthalenetrisulfonic acid, hexasodium salt

Following the procedure of Example 53, the amino product of Example 64 is treated with phosgene to produce the ureylene, the product of the Example.

EXAMPLE 66

8-[3-(3-Amino-p-toluamido)-p-toluenesulfonamido]--1,3,6-naphthalenetrisulfonic acid, trisodium salt

Following the procedure of Example 52 with 8-amino--1,3,6-naphthalenetrisulfonic acid, trisodium salt provides 8-(3-amino-p-toluenesulfonamido)-1,3,6-naphthalenetrisulfonic acid, trisodium salt. Reaction of the latter with 3-nitro-p-toluoyl chloride followed by reduction with palladium on carbon catalyst provides the product of the Example.

EXAMPLE 67

8,8'-[Ureylenebis[[[(4-methyl-3,1-phenylenecarbonyl)-imino](4-methyl-3,1-phenylenesulfonyl)]imino]]di--1,3,6-naphthalenetrisulfonic acid, hexasodium salt

Following the procedure of Example 53, the amino product of Example 66 is treated with phosgene to produce the ureylene, the product of the Example.

EXAMPLE 68

8-[3-(3-Aminobenzamido)-p-toluenesulfonamido]-
-1,3,6-naphthalenetrisulfonic acid, trisodium salt

Following the procedure of Example 52, reaction of 8-(3-amino-p-toluenesulfonamido)-1,3,6-naphthalenetrisulfonic acid, trisodium salt (Example 65) with 3-nitrobenzoyl chloride followed by reduction with palladium on carbon catalyst provides the product of the Example.

EXAMPLE 69

8,8'-[Ureylenebis[[(3,1-phenylenecarbonylimino)-
(4-methyl-3,1-phenylenesulfonyl)]imino]]di-1,3,6-
-naphthalenetrisulfonic acid, hexasodium salt

Following the procedure of Example 53, the amino product of Example 68 is treated with phosgene to produce the ureylene, the product of the Example.

EXAMPLE 70

8-[3-(3-Aminobenzamido)-p-toluenesulfonamido]-
-1,3,5-naphthalenetrisulfonic acid, trisodium salt

Following the procedure of Example 52, reaction of 8-(3-amino-p-toluenesulfonamido)-1,3,5-naphthalenetrisulfonic acid, trisodium salt with 3-nitrobenzoyl chloride followed by reduction with palladium on carbon catalyst provides the product of the Example.

EXAMPLE 71

8,8'-[Ureylenebis[[(3,1-phenylenecarbonylimino)-
(4-methyl-3,1-phenylenesulfonyl)]imino]]di-1,3,5-
-naphthalenetrisulfonic acid, hexasodium salt

Following the procedure of Example 53, the amino product of Example 70 is treated with phosgene to produce the ureylene, the product of the Example.

EXAMPLE 72

8-[5-(5-Amino-o-toluamido)-o-toluenesulfonamido]-
-1,3,5-naphthalenetrisulfonic acid, trisodium salt

Following the procedure of Example 52, reaction of 8-amino-1,3,5-naphthalenetrisulfonic acid with 5-nitro-o-toluenesulfonyl chloride provides 8-(5-nitro-o-toluenesulfonamido)-1,3,5-naphthalenetrisulfonic acid, trisodium salt,

followed by reduction with palladium on carbon catalyst provides 8-(5-amino-o-toluenesulfonamido)-1,3,5-naphthalenetrisulfonic acid, trisodium salt.

The preceding product is then reacted with 5-nitro-o-toluoyl chloride to yield 8-[5-(5-amino-o-toluamido)-o-toluenesulfonamido]-1,3,5-naphthalenetrisulfonic acid, trisodium salt.

EXAMPLE 73

8,8'-[Ureylenebis[[[(6-methyl-3,1-phenylenecarbonyl)-imino](6-methyl-3,1-phenylenesulfonyl)]imino]]di-1,3,5-naphthalenetrisulfonic acid, hexasodium salt

Following the procedure of Example 53, the amino product of Example 72 is treated with phosgene to produce the ureylene, the product of the Example.

EXAMPLE 74

8-[5-(5-Amino-o-toluamido)-o-toluenesulfonamido]-1,3,6-naphthalenetrisulfonic acid, trisodium salt

Following the procedure of Example 52, reaction of 8-amino-1,3,6-naphthalenetrisulfonic acid, with 5-nitro-o-toluenesulfonyl chloride provides 8-(5-nitro-o-toluenesulfonamido)-1,3,5-naphthalenetrisulfonic acid, trisodium salt, followed by reduction with palladium on carbon catalyst provides 8-(5-amino-o-toluenesulfonamido)-1,3,5-naphthalenetrisulfonic acid, trisodium salt.

The preceding product is then reacted with 5-nitro-o-toluoyl chloride to yield 8-[5-(5-amino-o-toluamido)-o-toluenesulfonamido]-1,3,5-naphthalenetrisulfonic acid, trisodium salt.

EXAMPLE 75

8,8'-[Ureylenebis[[[(6-methyl-3,1-phenylenecarbonyl)-imino](6-methyl-3,1-phenylenesulfonyl)]imino]]di-1,3,6-naphthalenetrisulfonic acid, hexasodium salt

Following the procedure of Example 53, the amino product of Example 74 is treated with phosgene to produce the ureylene, the product of the Example.

EXAMPLE 76

8-[N-(m-Aminobenzoyl)metanilamido]-1,3,5-naphthalenetrisulfonic acid, trisodium salt

Following the procedure of Example 52, reaction of 8-amino-1,3,5-naphthalenetrisulfonic acid, trisodium salt with 3-nitrobenzenesulfonyl chloride provides, after reduction, 8-(metanilamido)-1,3,5-naphthalenetrisulfonic acid, trisodium salt. Treatment with m-nitrobenzoyl chloride, reducing the product therefrom provides the product of the Example.

EXAMPLE 77

8,8'-[Ureylenebis[[(3,1-phenylenecarbonylimino)-3,1-phenylenesulfonyl]imino]]di-1,3,5-naphthalenetrisulfonic acid, hexasodium salt

Following the procedure of Example 53, treatment of the product of Example 76 with phosgene generates the ureylene, the product of the Example.

EXAMPLE 78

8-[N-(m-Aminobenzoyl)metanilamido]-1,3,6-naphthalenetrisulfonic acid, trisodium salt

Following the procedure of Example 52, reaction of 8-amino-1,3,6-naphthalenetrisulfonic acid, trisodium salt with 3-nitrobenzenesulfonyl chloride provides, after reduction, 8-(metanilamido)-1,3,6-naphthalenetrisulfonic acid, trisodium salt. Treatment with m-nitrobenzoyl chloride, reducing the product therefrom provides the product of the Example.

EXAMPLE 79

8,8'-[Ureylenebis[[(3,1-phenylenecarbonylimino)-3,1-phenylenesulfonyl]imino]]di-1,3,6-naphthalenetrisulfonic acid, hexasodium salt

Following the procedure of Example 53, treatment of the product of Example 78 with phosgene generates the ureylene, the product of the Example.

EXAMPLE 80

8-[3-(5-Amino-2,4-dimethylbenzamido)-p-toluenesulfonamido]-1,3,5-naphthalenetrisulfonic acid, trisodium salt

Following the procedure of Example 52, reaction of 8-(3-amino-p-toluenesulfonamido)-1,3,5-naphthalenetrisulfonic acid, trisodium salt with 5-nitro-2,4-dimethylbenzoyl chloride generates 8-[3-(5-nitro-2,4-dimethylbenzamido)-p-toluenesulfonamido]-1,3,5-naphthalenetrisulfonic acid, trisodium salt. Reduction with palladium on carbon catalyst gives the product of the Example.

EXAMPLE 81

8,8'-[Ureylenebis[[[(4,6-dimethyl-3,1-phenylenecarbonyl)imino](4-methyl-3,1-phenylenesulfonyl)]imino]]di-1,3,5-naphthalenetrisulfonic acid, hexasodium salt

Following the procedure of Example 53, phosgenation of the product from Example 80 provides the proudct of the Example.

EXAMPLE 82

8-[3-(5-Amino-2,4-dimethylbenzamido)-p-toluenesulfonamido]-1,3,6-naphthalenetrisulfonic acid, trisodium salt

Following the procedure of Example 52, reaction of 8-(3-amino-p-toluenesulfonamido)-1,3,6-naphthalenetrisulfonic acid, trisodium salt with 5-nitro-2,4-dimethylbenzoyl chloride generates 8-[3-(5-nitro-2,4-dimethylbenzamido)-p-toluenesulfonamido]-1,3,6-naphthalenetrisulfonic acid, trisodium salt. Reduction with palladium on carbon catalyst gives the product of the Example.

EXAMPLE 83

8,8'-[Ureylenebis[[[(4,6-dimethyl-3,1-phenylenecarbonyl)imino](4-methyl-3,1-phenylenesulfonyl)]imino]]di-1,3,6-naphthalenetrisulfonic acid, hexasodium salt

Following the procedure of Example 53, phosgenation of the product from Example 82 provides the product of the Example.

EXAMPLE 84

Preparation of Compressed Tablet

| Ingredient | mg/Tablet |
|---|---|
| Active Compound........................ | 0.5-500 |
| Dibasic Calcium Phosphate N.F. ......... | qs |
| Starch USP............................. | 40 |
| Modified Starch........................ | 10 |
| Magnesium Stearate USP................. | 1-5 |

EXAMPLE 85

Preparation of Compressed Tablet - Sustained Action

| Ingredient | mg/Tablet |
|---|---|
| Active Compound as Aluminum Lake*, Micronized............ | 0.5-500 (as acid equivalent) |
| Dibasic Calcium Phosphate N.F. ............ | qs |
| Alginic Acid.............................. | 20 |
| Starch USP................................ | 35 |
| Magnesium Stearate USP.................... | 1-10 |

*Complement inhibitor plus aluminum sulfate yields aluminum complement inhibitor. Complement inhibitor content in aluminum lake ranges from 5-30%.

EXAMPLE 86

Preparation of Hard Shell Capsule

| Ingredient | mg/Capsule |
|---|---|
| Active Compound................ | 0.5-500 |
| Lactose, Spray Dried.......... | qs |
| Magnesium Stearate............ | 1-10 |

EXAMPLE 87

Preparation of Oral Liquid (Syrup)

| Ingredient | % W/V |
|---|---|
| Active Compound.............. | 0.05-5 |
| Liquid Sugar................. | 75.0 |
| Methyl Paraben USP........... | 0.18 |
| Propyl Paraben USP........... | 0.02 |
| Flavoring Agent.............. | qs |
| Purified Water qs ad......... | 100.0 |

EXAMPLE 88

Preparation of Oral Liquid (Elixir)

| Ingredient | % W/V |
|---|---|
| Active Compound.............. | 0.05-5 |
| Alcohol USP................. | 12.5 |
| Glycerin USP................ | 45.0 |
| Syrup USP................... | 20.0 |
| Flavoring Agent............. | qs |
| Purified Water qs ad......... | 100.0 |

EXAMPLE 89

Preparation of Oral Suspension (Syrup)

| Ingredient | % W/V |
|---|---|
| Active Compound as Aluminum Lake, Micronized.......... | 0.05-5 (acid equivalent) |
| Polysorbate 80 USP................... | 0.1 |
| Magnesium Aluminum Silicate, Colloidal......................... | 0.3 |
| Flavoring Agent...................... | qs |
| Methyl Paraben USP................... | 0.18 |
| Propyl Paraben USP................... | 0.02 |
| Liquid Sugar......................... | 75.0 |
| Purified Water qs ad................. | 100.0 |

EXAMPLE 90

Preparation of Injectable Solution

| Ingredient | % W/V |
|---|---|
| Active Compound............. | 0.05-5 |
| Benzyl Alcohol N.F. ........ | 0.9 |
| Water for Injection......... | 100.0 |

EXAMPLE 91

Preparation of Injectable Oil

| Ingredient | % W/V |
|---|---|
| Active Compound......... | 0.05-5 |
| Benzyl Alcohol.......... | 1.5 |
| Sesame Oil qs ad........ | 100.0 |

EXAMPLE 92

Preparation of Intra-Articular Product

| Ingredient | Amount |
|---|---|
| Active Compound........................ | 2-20 mg |
| NaCl (physiological saline)........... | 0.9% |
| Benzyl Alcohol........................ | 0.9% |
| Sodium Carboxymethylcellulose......... | 1-5% |
| pH adjusted to 5.0-7.5 | |
| Water for Injection qs ad............. | 100% |

EXAMPLE 93

Preparation of Injectable Depo Suspension

| Ingredient | % W/V |
|---|---|
| Active Compound....................... | 0.05-5 (acid equivalent) |
| Polysorbate 80 USP.................... | 0.2 |
| Polyethylene Glycol 4000 USP.......... | 3.0 |
| Sodium Chloride USP................... | 0.8 |
| Benzyl Alcohol N.F. .................. | 0.9 |
| HCl to pH 6-8......................... | qs |
| Water for Injection qs ad............. | 100.0 |

EXAMPLE 94

Preparation of Dental Paste

| Ingredient | % W/W |
|---|---|
| Active Compound....................... | 0.05-5 |
| Zinc Oxide............................ | 15 |
| Polyethylene Glycol 4000 USP.......... | 50 |
| Distilled Water qs.................... | 100 |

EXAMPLE 95

Preparation of Dental Ointment

| Ingredient | % W/W |
|---|---|
| Active Compound....................... | 0.05-5 |
| Petrolatum, White USP qs.............. | 100 |

EXAMPLE 96

Preparation of Dental Cream

| Ingredient | % W/W |
|---|---|
| Active Compound....................... | 0.05-5 |
| Mineral Oil........................... | 50 |

EXAMPLE 96 (Cont'd)

Preparation of Dental Cream

| Ingredient | % W/W |
| --- | --- |
| Beeswax | 15 |
| Sorbitan Monostearate | 2 |
| Polyoxyethylene 20 Sorbitan Monostearate | 3 |
| Methyl Paraben USP | 0.18 |
| Propyl Paraben USP | 0.02 |
| Distilled Water qs | 100 |

EXAMPLE 97

Preparation of Topical Cream

| Ingredient | % W/W |
| --- | --- |
| Active Compound | 0.05-5 |
| Sodium Lauryl Sulfate | 1 |
| Propylene Glycol | 12 |
| Stearyl Alcohol | 25 |
| Petrolatum, White USP | 25 |
| Methyl Paraben USP | 0.18 |
| Propyl Paraben USP | 0.02 |
| Purified Water qs | 100 |

EXAMPLE 98

Preparation of Topical Ointment

| Ingredient | % W/W |
| --- | --- |
| Active Compound | 0.05-5 |
| Cholesterol | 3 |
| Stearyl Alcohol | 3 |
| White Wax | 8 |
| Petrolatum, White USP qs | 100 |

EXAMPLE 99

Preparation of Spray Lotion (non-Aerosol)

| Ingredient | % W/W |
| --- | --- |
| Active Compound | 0.05-5 |
| Isopropyl Myristate | 20 |
| Alcohol (Denatured) qs | 100 |

EXAMPLE 100

Preparation of Buccal Tablet

| Ingredient | g/Tablet |
| --- | --- |
| Active Ingredient | 0.00325 |
| 6 x Sugar | 0.29060 |
| Acacia | 0.01453 |
| Soluble Starch | 0.01453 |
| F. D. & C. Yellow No. 6 Dye | 0.00049 |
| Magnesium Stearate | 0.00160 |
| | 0.32500 |

The final tablet will weigh about 325 mg. and may be compressed into buccal tablets in flat faced or any other tooling shape convenient for buccal administration.

EXAMPLE 101

Preparation of Lozenge

| Ingredient | g/Lozenge |
| --- | --- |
| Active Ingredient | 0.0140 |
| Kompact® Sugar (Sucrest Co.) | 0.7138 |
| 6 x Sugar | 0.4802 |
| Sorbitol (USP Crystalline) | 0.1038 |
| Flavor | 0.0840 |
| Magnesium Stearate | 0.0021 |
| Dye | qs |
| Stearic Acid | 0.0021 |
| | 1.4000 |

The ingredients are compressed into 5/8" flat based lozenge tooling. Other shapes may also be utilized.

26,913



CLAIMS:

1. A compound of the formula:

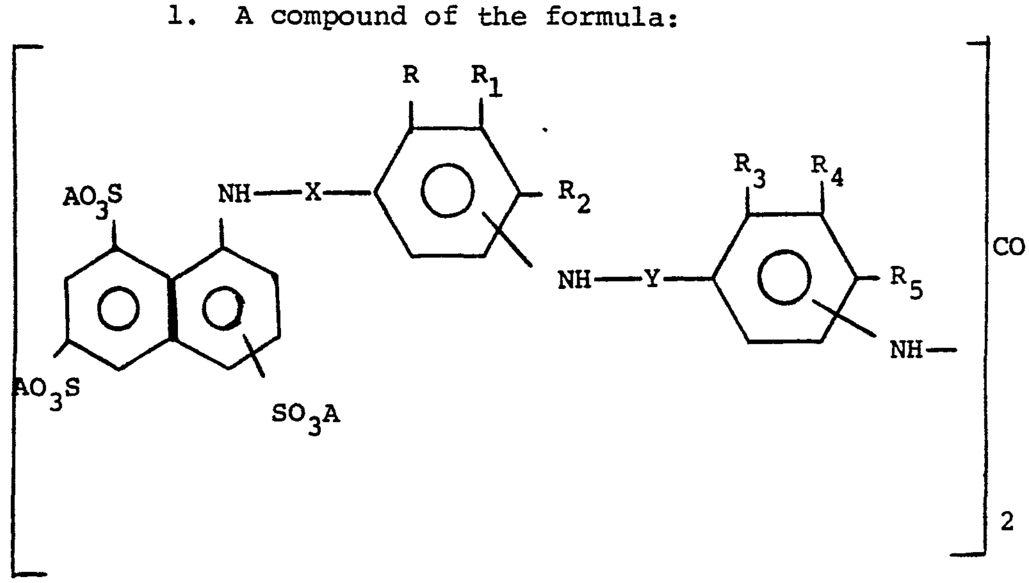

wherein X and Y are each selected from the group consisting of -CO- and $-SO_2-$; R and $R_3$ are each selected from the group consisting of hydrogen, methyl and $-SO_3A$, wherein A is a pharmaceutically acceptable salt cation; $R_1$ and $R_4$ are each selected from the group consisting of hydrogen and -COOB, wherein B is selected from the group consisting of hydrogen and a pharmaceutically acceptable salt cation; $R_2$ and $R_5$ are each selected from the group consisting of hydrogen and methyl; with the proviso that R, $R_1$, $R_2$, $R_3$, $R_4$ and $R_5$ may not all be hydrogen; with the second proviso that when X and Y are both -CO- and $R_2$ is methyl, then R, $R_1$, $R_3$, $R_4$ and $R_5$ may not all be hydrogen; and with the third proviso that no phenyl moiety can contain both $-SO_3A$ and -COOB.

2. A compound according to Claim 1 wherein either $R_1$ or $R_4$, or both, are -COOB; and R and $R_3$ are hydrogen.

3. A compound according to Claim 1 wherein either R or $R_3$, or both, are $-SO_3A$; and $R_1$ and $R_4$ are hydrogen.

4. A compound according to Claim 1, selected from 8,8'-[ureylenebis[(4-methyl-3,1-phenylenecarbonyl)imino (4-methyl-3,1-phenylenecarbonyl)imino]]di-1,3,5-naphthalenetrisulfonic acid, hexasodium salt,
8,8'-[ureylenebis[(4-methyl-3,1-phenylenesulfonyl)imino (4-methyl-3,1-phenylenecarbonyl)imino]]di-1,3,5-naphthalenetrisulfonic acid, hexasodium salt,
8,8'-[ureylenebis[(4-methyl-3,1-phenylenesulfonyl)imino (4-methyl-3,1-phenylenecarbonyl)imino]]di-1,3,5-naphthalenetrisulfonic acid, hexasodium salt,
8,8'-ureylenebis[( 6-dimethyl-3,1-phenylenesulfonyl)imino (4-methyl-3,1-phenylenecarbonyl)imino]]di-1,3,5-naphthalenetrisulfonic acid, hexasodium salt,
8,8'-[ureylenebis[(4-methyl-3,1-phenylenesulfonyl)imino-3,1--phenylenecarbonylimino]]di-1,3,5-naphthalenetrisulfonic acid, hexasodium salt,
5,5"-ureylenebis[2'-methyl-5'-(4,6,8-trisulfo-1-naphthylcarbamoyl)isophthalanilic acid], octasodium salt,
5,5"-ureylenebis[2'-methyl-5'-(4,6,8-trisulfo-1-naphthylcarbamoyl)isophthalanilic acid), hexasodium salt,
5,5'-[ureylenebis[(4-methyl-3,1-phenylenecarbonyl)imino]bis [N-(4,6,8-trisulfo-1-naphthyl)-isophthalamic acid], octasodium salt,
5,5'-ureylenebis[(4-methyl-3,1-phenylenecarbonyl)imino]bis [N-(4,6,8-trisulfo-1-naphthyl)-isophthalamic acid]hexasodium salt,
8,8'-[ureylenebis[(2-sulfo-4,1-phenylenecarbonyl)imino(4-methyl--3,1-phenylenecarbonyl)imino]]di-1,3,5-naphthalenetrisulfonic acid, octasodium salt,
5,5"-ureylenebis[2'-methyl-5'-(3,6,8-trisulfo-1-naphthylcarbamoyl)isophthalanilic acid], octasodium salt,
5,5'-[ureylenebis[(4-methyl-3,1-phenylenecarbonyl)imino]]-bis [N-(3,6,8-trisulfo-1-naphthyl)isophthalamic acid], octasodium salt,
8,8'-[ureylenebis[(2-sulfo-4,1-phenylenecarbonyl)imino(4--imino]]di-1,3,6-naphthalenetrisulfonic acid, octasodium salt,
8,8'-[ureylenebis[(2-sulfo-4,1-phenylenecarbonyl)imino](2--sulfo-4,1-phenylenecarbonyl)imino]]di-1,3,6-naphthalenetrisulfonic acid, decasodium salt,

8,8'-[ureylenebis[(2-sulfo-4,1-phenylenecarbonyl)imino(2--sulfo-4,1-phenylenecarbonyl)imino]]di-1,3,5-naphthalenetrisulfonic acid, decasodium salt,
5,5'-[ureylenebis[(2-sulfo-4,1-phenylenecarbonyl)imino]]bis [N-4,6,8-trisulfo-1-naphthylisophthalamic acid], octasodium salt,
5,5'-ureylenebis[N-[3-carboxy-5-(4,6,8-trisulfo-1-naphthylcarbamoyl)phenyl]isophthalamic acid], decasodium salt,
5,5'-ureylenebis[N-[3-carboxy-5-(4,6,8-trisulfo-1-naphthylcarbamoyl)phenyl]isophthalamic acid], hexasodium salt,
5,5'-ureylenebis[N-[3-carboxy-5-(3,6,8-trisulfo-1-naphthylcarbamoyl)phenyl]isophthalamic acid], decasodium salt,
5,5"-ureylenebis[3'-sulfo-4'-(4,6,8-trisulfo-1-naphthylcarbamoyl)isophthalanilic acid], decasodium salt,
8,8'-[ureylenebis[(2-sulfo-4,1-phenylenecarbonyl)-imino(6--methyl-3,1-phenylenesulfonyl)imino]]di-1,3,6-naphthalenetrisulfonic acid, octasodium salt,
8,8'-[ureylenebis[(2-sulfo-4,1-phenylenecarbonyl)imino(6--methyl-3,1-phenylenesulfonyl)imino]]di-1,3,5-naphthalenetrisulfonic acid, octasodium salt,
8,8'-[ureylenebis[(2-sulfo-4,1-phenylenecarbonyl)imino(4,6-dimethyl-3,1-phenylenesulfonyl)imino]]di-1,3,6-naphthalenetrisulfonic acid, octasodium salt,
8,8'-[ureylenebis[(2-sulfo-4,1-phenylenecarbonyl)imino(4,6--dimethyl-3,1-phenylenesulfonyl)imino]]-di-1,3,5-naphthalenetrisulfonic acid, octasodium salt,
5,5"-ureylenebis[2'-methyl-5'-(4,6,8-trisulfo-1-naphthylsulfamoyl)isophthalanilic acid], octasodium salt,
5,5"-ureylenebis[2'-methyl-5'- (3,6,8-trisulfo-1-naphthylsulfamoyl)isophthalanilic acid], octasodium salt,
8,8'-[ureylenebis[(2-sulfo-1,4-phenylenecarbonyl)imino(3,1--phenylenesulfonyl)imino]]di-1,3,6-naphthalenetrisulfonic acid, octasodium salt,
8,8'-[ureylenebis[[[(4-methyl-3,1-phenylenecarbonyl)-imino](4-methyl-3,1-phenylenesulfonyl)]imino]]di-1,3,5-naphthalenetrisulfonic acid, hexasodium salt,
8,8'-[ureylenebis[[[(4-methyl-3,1-phenylenecarbonyl)-imino]

(4-methyl-3,1-phenylenesulfonyl)imino]]di-1,3,6-naphthalenetrisulfonic acid, hexasodium salt,

8,8'-[ureylenebis[[(3,1-phenylenecarbonylimino)-(4-methyl--3,1-phenylenesulfonyl)]imino]]di-1,3,6-naphthalenetrisulfonic acid, hexasodium salt,

8,8'-[ureylenebis[[(3,1-phenylenecarbonylimino)-(4-methyl--3,1-phenylenesulfonyl)]imino]]di-1,3,5-naphthalenetrisulfonic acid, hexasodium salt,

8,8'-[ureylenebis[[[(6-methyl-3,1-phenylenecarbonyl)-imino](6-methyl-3,1-phenylenesulfonyl)]imino]]di-1,3,5-naphthalenetrisulfonic acid, hexasodium salt,

8,8'-[ureylenebis[[[(6-methyl-3,1-phenylenecarbonyl)-imino](6-methyl-3,1-phenylenesulfonyl)]imino]]di-1,3,6-naphthalenetrisulfonic acid, hexasodium salt,

8,8'-[ureylenebis[[(3,1-phenylenecarbonylimino)-3,1-phenylene-sulfonyl]imino]]di-1,3,5-naphthalenetrisulfonic acid, hexasodium salt,

8,8'-[ureylenebis[[(3,1-phenylenecarbonylimino)-3,1-phenylene-sulfonyl]imino]]di-1,3,6-naphthalenetrisulfonic acid, hexasodium salt,

8,8'-[ureylenebis[[[(4,6-dimethyl-3,1-phenylenecarbonyl)imino](4-methyl-3,1-phenylenesulfonyl)]-imino]]di-1,3,5-naphthalenetrisulfonic acid, hexasodium salt,

8,8'-[ureylenebis[[[(4,6-dimethyl-3,1-phenylenecarbonyl)imino](4-methyl-3,1-phenylenesulfonyl)]imino]]di-1,3,6-naphthalenetrisulfonic acid, hexasodium salt,

8,8'-[ureylenebis[(m-phenylenesulfonylimino) (4-methyl-3,1--phenylenesulfonyl)imino]]di-1,3,5-naphthalenetrisulfonic acid, hexasodium salt,

8,8'-[ureylenebis[(m-phenylenesulfonylimino)(4-methyl-3,1--phenylsulfonyl)imino]]di-1,3,6-naphthalenetrisulfonic acid, hexasodium salt,

8,8'-[ureylenebis[[(1,3-phenylenesulfonylimino)-1,3-phenylene-sulfonyl]imino]]di-1,3,6-naphthalenetrisulfonic acid, hexasodium salt,

8,8'-[ureylenebis]](1,3-phenylenesulfonylimino)-1,3-phenylene-sulfonyl]imino]]di-1,3,5-naphthalenetrisulfonic acid, hexa-

0008154



sodium salt,
8,8'-[ureylenebis[[[(6-methyl-3,1-phenylene)sulfonyl]imino]-[[(6-methyl-3,1-phenylene)sulfonyl]imino]]]di-1,3,6-naphthalenetrisulfonic acid, hexasodium salt, and
8,8'-[ureylenebis[[[(6-methyl-3,1-phenylene)sulfonyl]imino]-[[(6-methyl-3,1-phenylene)sulfonyl]imino]]]di-1,3,5-naphthalenetrisulfonic acid, hexasodium salt.

5. A compound of the formula

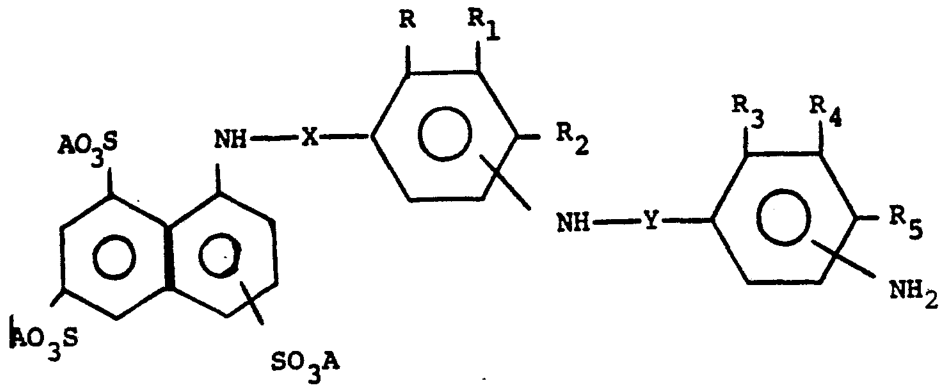

wherein X and Y are each selected from the group consisting of -CO- and $-SO_2-$; R and $R_3$ are each selected from the group consisting of hydrogen, methyl and $-SO_3A$, wherein A is a pharmaceutically acceptable salt cation; $R_1$ and $R_4$ are each selected from the group consisting of hydrogen and -COOB, wherein B is selected from the group consisting of hydrogen and a pharmaceutically acceptable salt cation; $R_2$ and $R_5$ are each selected from the group consisting of hydrogen and methyl; with the proviso that R, $R_1$, $R_2$, $R_3$, $R_4$ and $R_5$ may not all be hydrogen; with the second proviso that when X and Y are both -CO- and $R_2$ is methyl, then R, $R_1$, $R_3$, $R_4$ and $R_5$ may not all be hydrogen; and with the third proviso that no phenyl moiety can contain both $-SO_3A$ and -COOB.

6. A compound according to Claim 5, selected from
5-amino-2'-methyl-5'-(4,6,8-trisulfo-1-naphthylcarbamoyl)isophthalanilic acid, trisodium salt,
5-(3-amino-p-toluamido)-N-(4,6,8-trisulfo-1-naphthyl)isophthalamic acid, trisodium salt,
8-[3-(4-amino-2-sulfobenzamido)-p-toluamido]-1,3,5-naphthalenetrisulfonic acid, tetrasodium salt,

5-amino-2'-methyl-5'-(3,6,8-trisulfo-1-naphthylcarbamoyl)isophthalanilic acid, trisodium salt,
5-(3-amino-p-toluamido)-N-(3,6,8-trisulfo-1-naphthyl)isophthalamic acid, trisodium salt,
8-[3-(4-amino-2-sulfobenzamido)-p-toluamido]-1,3,6-naphthalenetrisulfonic acid, tetrasodium salt,
8-[4-(4-amino-2-sulfobenzamido)-2-sulfobenzamido)-1,3,6-naphthalenetrisulfonic acid, pentasodium salt,
8-[4-(4-amino-2-sulfobenzamido)-2-sulfobenzamido]-1,3,5-naphthalenetrisulfonic acid, pentasodium salt,
5-(4-amino-2-sulfobenzamido)-N-(4,6,8-trisulfo-1-naphthyl) isophthalamic acid, tetrasodium salt,
5-amino-3'-carboxy-5'-(4,6,8-trisulfo-1-naphthylcarbamoyl) isophthalanilic acid, trisodium salt,
5-amino-3'-carboxy-5'-(3,6,8-trisulfo-1-naphthylcarbamoyl) isophthalanilic acid, trisodium salt,
5-amino-3'-sulfo-4'-(4,6,8-trisulfo-1-naphthylcarbamoyl) isophthalanilic acid, tetrasodium salt,
8-[5-(4-amino-2-sulfobenzamido)-o-toluenesulfonamido]-1,3,6-naphthalenetrisulfonic acid, tetrasodium salt,
8-[5-(4-amino-2-sulfobenzamido)-o-toluenesulfonamido]-1,3,5-naphthalenetrisulfonic acid, tetrasodium salt,
8-[5-(4-amino-2-sulfobenzamido)-2,4-xylenesulfonamido]-1,3,6-naphthalenetrisulfonic acid, tetrasodium salt,
8-[5-(4-amino-2-sulfobenzamido)-2,4-xylenesulfonamido]-1,3,5-naphthalenetrisulfonic acid, tetrasodium salt,
5-amino-2'-methyl-5'-(4,6,8-trisulfo-1-naphthylsulfamoyl)-isophthalanilic acid, trisodium salt,
5-amino-2'-methyl-5'-(3,6,8-trisulfo-1-naphthylsulfamoyl)-isophthalanilic acid, trisodium salt,
8-[$N^3$-(4-amino-2-sulfobenzoyl)metanilamido]-1,3,6-naphthalenetrisulfonic acid, tetrasodium salt,
8-[3-(3-amino-p-toluamido)-p-toluenesulfonamido]-1,3,5-naphehalenetrisulfonic acid, trisodium salt,
8-[3-(3-amino-p-toluamido)-p-toluenesulfonamido]-1,3,6-naphthalenetrisulfonic acid, trisodium salt,

8-[3-(3-aminobenzamido)-p-toluenesulfonamido]-1,3,6-naphthalenetrisulfonic acid, trisodium salt,
8-[3-(3-aminobenzamido)-p-toluenesulfonamido]-1,3,5-naphthalenetrisulfonic acid, trisodium salt,
8-[5-(5-amino-o-toluamido)-o-toluenesulfonamido]-1,3,5-naphthalenetrisulfonic acid, trisodium salt,
8-[5-(5-amino-o-toluamido)-o-toluenesulfonamido]-1,3,6-naphthalenetrisulfonic acid, trisodium salt,
8-[N-(m-aminobenzoyl)metanilamido]-1,3,5-naphthalenetrisulfonic acid, trisodium salt,
8-[N-(m-aminobenzoyl)metanilamido]-1,3,6-naphthalenetrisulfonic acid, trisodium salt,
8-[3-(5-amino-2,4-dimethylbenzamido)-p-toluenesulfonamido]-1,3,5-naphthalenetrisulfonic acid, trisodium salt,
8-[3-(5-amino-2,4-dimethylbenzamido)-p-toluenesulfonamido]-1,3,6-naphthalenetrisulfonic acid, trisodium salt,
8-(3-metanilamido-p-toluenesulfonamido)-1,3,5-naphthalenetrisulfonic acid, trisodium salt,
8-(3-metanilamido-p-toluenesulfonamido)-1,3,6-naphthalenetrisulfonic acid, trisodium salt,
8-[$N^3$-(m-aminophenylsulfonyl)metanilamido]-1,3,6-naphthalenetrisulfonic acid, trisodium salt,
8-[$N^3$-(m-aminophenylsulfonyl)metanilamido]-1,3,5-naphthalenetrisulfonic acid, trisodium salt,
8-[5-(5-amino-o-toluenesulfonamido)-o-toluenesulfonamido]-1,3,6-naphthalenetrisulfonic acid, trisodium salt, and
8-[5-(5-amino-o-toluenesulfonamido)-o-toluenesulfonamido]-1,3,5-naphthalenetrisulfonic acid, trisodium salt.

7. A method of inhibiting the complement system in a body fluid which comprises subjecting said body fluid to the action of an effective complement inhibiting amount of a compound of the formula:

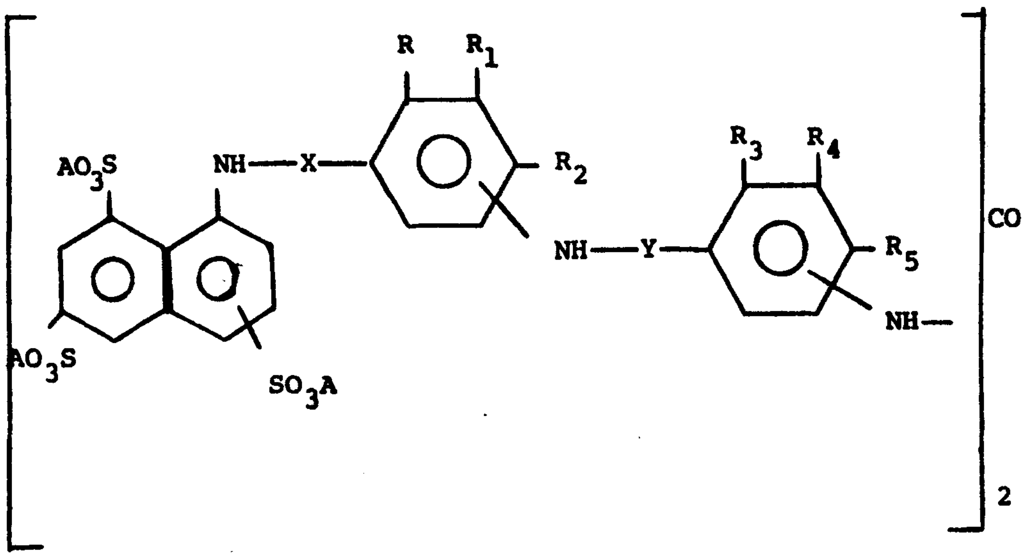

wherein X and Y are each selected from the group consisting of -CO- and $-SO_2-$; R and $R_3$ are each selected from the group consisting of hydrogen, methyl and $-SO_3A$, wherein A is a pharmaceutically acceptable salt cation; $R_1$ and $R_4$ are each selected from the group consisting of hydrogen and -COOB, wherein B is selected from the group consisting of hydrogen and a pharmaceutically acceptable salt cation, $R_2$ and $R_5$ are each selected from the group consisting of hydrogen and methyl; with the proviso that R, $R_1$, $R_2$, $R_3$, $R_4$ and $R_5$ may not all be hydrogen; with the second proviso that when X and Y are both -CO- and $R_2$ is methyl, then R, $R_1$, $R_3$, $R_4$ and $R_5$ may not all be hydrogen; and with the third proviso that no phenyl moiety can contain both $-SO_3A$ and -COOB.

8. A method according to Claim 7 wherein the body fluid is blood serum.

9. A method according to Claim 7 wherein the compound is selected from 8,8'-[ureylenebis[(4-methyl-3,1--phenylenecarbonyl)imino(4-methyl-3,1-phenylenecarbonyl)imino]]di-1,3,5-naphthalenetrisulfonic acid, hexasodium salt,
8,8'-[ureylenebis[(4-methyl-3,1-phenylenesulfonyl)imino(4--methyl-3,1-phenylenecarbonyl)imino]]di-1,3,5-naphthalenetrisulfonic acid, hexasodium salt,
8,8'-[ureylenebis[(6-methyl-3,1-phenylenesulfonyl)imino(4--methyl-3,1-phenylenecarbonyl)imino]]di-1,3,5-naphthalenetrisulfonic acid, hexasodium salt,
8,8'-[ureylenebis[(4,6-dimethyl-3,1-phenylenesulfonyl)imino (4-methyl-3,1-phenylenecarbonyl)imino]]di-1,3,5-naphthalenetrisulfonic acid, hexasodium salt,
8,8'-[ureylenebis[(4-methyl-3,1-phenylenesulfonyl)imino-3,1--phenylenecarbonylimino]]di-1,3,5-naphthalenetrisulfonic acid, hexasodium salt,
5,5"-ureylenebis[2'-methyl-5'-(4,6,8-trisulfo-1-naphthylcarbamoyl)isophthalanilic acid], octasodium salt,
5,5"-ureylenebis[2'-methyl-5'-(4,6,8-trisulfo-1-naphthylcarbamoyl)isophthalanilic acid), hexasodium salt,
5,5'-[ureylenebis[(4-methyl-3,1-phenylenecarbonyl)imino]bis (N-(4,6,8-trisulfo-1-naphthyl)-isophthalamic acid], octasodium salt,
5,5'-ureylenebis[(4-methyl-3,1-phenylenecarbonyl)imino]bis [N-4,6,8-trisulfo-1-naphthyl)-isophthalamic acid], hexasodium salt,
8,8'-[ureylenebis[(2-sulfo-4,1-phenylenecarbonyl)imino(4--methyl-3,1-phenylenecarbonyl)imino]]di-1,3,5-naphthalenetrisulfonic acid, octasodium salt,
5,5"-ureylenebis[2'-methyl-5'-(3,6,8-trisulfo-1-naphthylcarbamoyl)isophthalanilic acid], octasodium salt,
5,5'-[ureylenebis[(4-methyl-3,1-phenylenecarbonyl)imino]]-bis [N-(3,6,8-trisulfo-1-naphthyl)isophthalamic acid], octasodium salt,
8,8'-[ureylenebis[(2-sulfo-4,1-phenylenecarbonyl)imino(4--methyl-3,1-phenylenecarbonyl)imino]]-di-1,3,6-naphthalenetrisulfonic acid, octasodium salt,
8,8'-[ureylenebis[(2-sulfo-4,1-phenylenecarbonyl)imino](2--sulfo-4,1-phenylenecarbonyl)imino]]di-1,3,6-naphthalenetrisulfonic acid, decasodium salt,
8,8'-[ureylenebis[(2-sulfo-4,1-phenylenecarbonyl)imino(2--sulfo-4,1-phenylenecarbonyl)imino]]di-1,3,5-naphthalenetrisulfonic acid, decasodium salt,
5,5'-[ureylenebis[(2-sulfo-4,1-phenylenecarbonyl)imino]]bis [N-4,6,8-trisulfo-1-naphthylisophthalamic acid], octasodium salt,

5,5'-ureylenebis[N-[3-carboxy-5-(4,6,8-trisulfo-1-naphthylcarbamoyl)phenyl]isophthalamic acid], decasodium salt,
5,5'-ureylenebis[N-[3-carboxy-5-(4,6,8-trisulfo-1-naphthylcarbamoyl)phenyl]isophthalamic acid], hexasodium salt,
5,5'-ureylenebis[N-[3-carboxy-5-(3,6,8-trisulfo-1-naphthylcarbamoyl)phenyl]isophthalamic acid], decasodium salt,
5,5"-ureylenebis[3'-sulfo-4'-(4,6,8-trisulfo-1-naphthylcarbamoyl)isophthalanilic acid], decasodium salt,
8,8'-[ureylenebis[(2-sulfo-4,1-phenylenecarbonyl)-imino(6--methyl-3,1-phenylenesulfonyl)imino]]di-1,3,6-naphthalenetrisulfonic acid, octasodium salt,
8,8'-[ureylenebis[(2-sulfo-4,1-phenylenecarbonyl)imino(6--methyl-3,1-phenylenesulfonyl)imino]]di-1,3,5-naphthalenetrisulfonic acid, octasodium salt,
8,8'-[ureylenebis[(2-sulfo-4,1-phenylenecarbonyl)imino(4,6--dimethyl-3,1-phenylenesulfonyl)imino]]di-1,3,6-naphthalenetrisulfonic acid, octasodium salt,
8,8'-[ureylenebis[(2-sulfo-4,1-phenylenecarbonyl)imino(4,6--dimethyl-3,1-phenylenesulfonyl)imino]]di-1,3,5-naphthalenetrisulfonic acid, octasodium salt,
5,5"-ureylenebis[2'-methyl-5'-(4,6,8-trisulfo-1-naphthylsulfamoyl)isophthalanilic acid], octasodium salt,
5,5"-ureylenebis[2'-methyl-5'-(3,6,8-trisulfo-1-naphthylsulfamoyl)isophthalanilic acid], octasodium salt,
8,8'-[ureylenebis[(2-sulfo-1,4-phenylenecarbonyl)imino(3,1--phenylenesulfonyl)imino]]di-1,3,6-naphthalenetrisulfonic acid, octasodium salt,
8,8'-[ureylenebis[[[(4-methyl-3,1-phenylenecarbonyl)-imino](4-methyl-3,1-phenylenesulfonyl)]imino]]di-1,3,5-naphthalenetrisulfonic acid, hexasodium salt,
8,8'-[ureylenebis[[[(4-methyl-3,1-phenylenecarbonyl)-imino](4-methyl-3,1-phenylenesulfonyl)]imino]]di-1,3,6-naphthalenetrisulfonic acid, hexasodium salt,
8,8'-[ureylenebis[[(3,1-phenylenecarbonylimino)-(4-methyl--3,1-phenylenesulfonyl)]imino]]di-1,3,6-naphthalenetrisulfonic acid, hexasodium salt,

8,8'-[ureylenebis[[(3,1-phenylenecarbonylimino)-(4-methyl--3,1-phenylenesulfonyl)]imino]]di-1,3,5-naphthalenetrisulfonic acid, hexasodium salt,
8,8'-[ureylenebis[[[(6-methyl-3,1-phenylenecarbonyl)-imino](6-methyl-3,1-phenylenesulfonyl)]imino]]di-1,3,5-naphthalenetrisulfonic acid, hexasodium salt,
8,8'-[ureylenebis[[[(6-methyl-3,1-phenylenecarbonyl)-imino](6-methyl-3,1-phenylenesulfonyl)]imino]]di-1,3,6-naphthalenetrisulfonic acid, hexasodium salt.
8,8'-[ureylenebis[[(3,1-phenylenecarbonylimino)-3,1-phenylenesulfonyl]imino]]di-1,3,5-naphthalenetrisulfonic acid, hexasodium salt,
8,8'-[ureylenebis[[(3,1-phenylenecarbonylimino)-3,1-phenylenesulfonyl]imino]]di-1,3,6-naphthalenetrisulfonic acid, hexasodium salt,
8,8'-[ureylenebis[[[(4,6-dimethyl-3,1-phenylenecarbonyl)imino](4-methyl-3,1-phenylenesulfonyl)]-imino]]di-1,3,5-naphthalenetrisulfonic acid, hexasodium salt,
8,8'-[ureylenebis[[[(4,6-dimethyl-3,1-phenylenecarbonyl)imino](4-methyl-3,1-phenylenesulfonyl)]imino]]di-1,3,6-naphthalenetrisulfonic acid, hexasodium salt,
8,8'-[ureylenebis[(m-phenylenesulfonylimino)(4-methyl-3,1--phenylenesulfonyl)imino]]di-1,3,5-naphthalenetrisulfonic acid, hexasodium salt,
8,8'-[ureylenebis[(m-phenylenesulfonylimino)(4-methyl-3,1--phenylsulfonyl)imino]]di-1,3,6-naphthalenetrisulfonic acid, hexasodium salt,
8,8'-[ureylenebis[[(1,3-phenylenesulfonylimino)-1,3-phenylenesulfonyl]imino]]di-1,3,6-naphthalenetrisulfonic acid, hexasodium salt,
8,8'-[ureylenebis[[(1,3-phenylenesulfonylimino)-1,3-phenylenesulfonyl]imino]]di-1,3,5-naphthalenetrisulfonic acid, hexasodium salt,
8,8'-[ureylenebis[[[(6-methyl-3,1-phenylene)sulfonyl]imino]--[[(6-methyl-3,1-phenylene)sulfonyl]imino]]]di-1,3,6-naphthalenetrisulfonic acid, hexasodium salt, and
8,8'-[ureylenebis[[[(6-methyl-3,1-phenylene)sulfonyl]imino]-

-[[(6-methyl-3,1-phenylene)sulfonyl]imino]]]di-1,3,5-naphthalenetrisulfonic acid, hexasodium salt.

10. A method for the preparation of a compound of the formula:

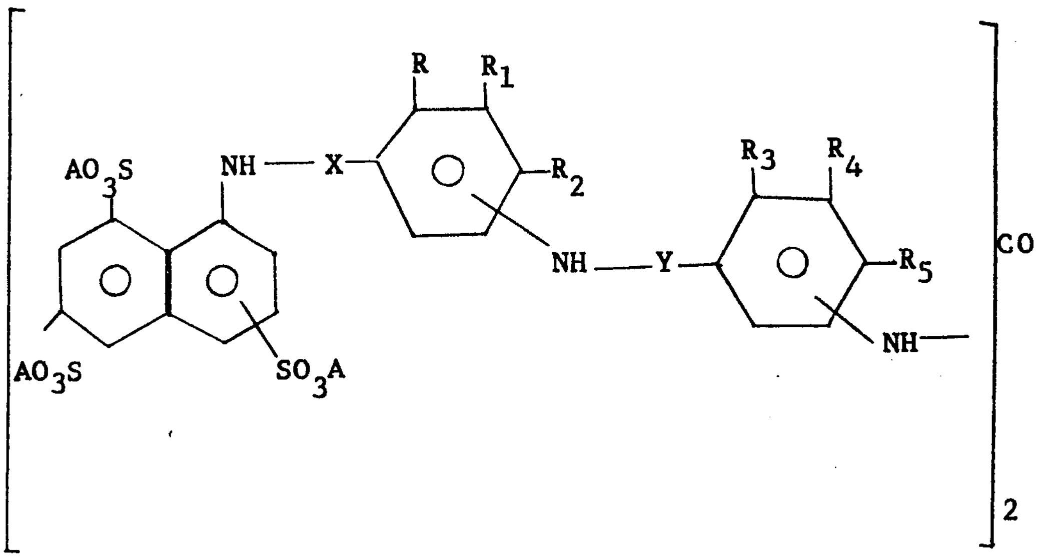

wherein X and Y are each selected from the group consisting of -CO- and $-SO_2-$; R and $R_3$ are each selected from the group consisting of hydrogen, methyl and $-SO_3A$, wherein A is a pharmaceutically acceptable salt cation; $R_1$ and $R_4$ are each selected from the group consisting of hydrogen and -COOB, wherein B is selected from the group consisting of hydrogen and a pharmaceutically acceptable salt cation; $R_2$ and $R_5$ are each selected from the group consisting of hydrogen and methyl; with the proviso that R, $R_1$, $R_2$, $R_3$, $R_4$ and $R_5$ may not all be hydrogen; with the second proviso that when X and Y are both -CO- and $R_2$ is methyl, then R, $R_1$,$R_3$ $R_4$ and $R_5$ may not all be hydrogen; and with the third proviso

that no phenyl moiety can contain both $-SO_3A$ and -COOB; which comprises bubbling phosgene gas into an aqueous solution of an amino substituted-phenylenecarbonyl(and sulfonyl) imino-substituted-phenylenecarbonyl(and sulfonyl)imino naphthalenetrisulfonic acid, trialkali metal salt in the presence of alkali metal carbonate or pyridine until acidic to Congo Red indicator, neutralization with alkali metal carbonate or pyridine and precipitation of the product from aqueous solution with alcohol.

TREGEAR, THIEMANN & BLEACH,
CHARTERED PATENT AGENTS,
ENTERPRISE HOUSE,
ISAMBARD BRUNEL ROAD,
PORTSMOUTH PO1 2AN
AND
49/51, BEDFORD ROW, LONDON, WC1V 6RL

0008154

European Patent Office

# EUROPEAN SEARCH REPORT

Application number

EP 79 301 036.4

| Category | DOCUMENTS CONSIDERED TO BE RELEVANT: Citation of document with indication, where appropriate, of relevant passages | Relevant to claim | CLASSIFICATION OF THE APPLICATION (Int. Cl.3) |
|---|---|---|---|
| D,A | THE BIOCHEMICAL JOURNAL, Volume 47, 1950 Cambridge E.D. WILLS et al. "Studies on Suramin. 9. The action of the drug on some enzymes" pages 158 to 170<br>-- | | C 07 C 143/675<br>C 07 C 143/80<br>A 61 K 31/18<br>A 61 K 31/185//<br>C 07 C 79/46<br>C 07 C 143/55<br>C 07 C 143/70 |
| A | US - A - 4 051 176 (S. BERNSTEIN et al.)<br>---- | | TECHNICAL FIELDS SEARCHED (Int.Cl.3)<br>A 61 K 31/00<br>C 07 C 143/00 |

CATEGORY OF CITED DOCUMENTS

X: particularly relevant
A: technological background
O: non-written disclosure
P: intermediate document
T: theory or principle underlying the invention
E: conflicting application
D: document cited in the application
L: citation for other reasons

&: member of the same patent family, corresponding document

X The present search report has been drawn up for all claims

| Place of search | Date of completion of the search | Examiner |
|---|---|---|
| Berlin | 08-11-1979 | STOOS |

EPO Form 1503.1 06.78